# EUROPEAN PATENT APPLICATION

(11) **EP 4 403 567 A1**
(43) Date of publication of application: **24.07.2024**
(21) Application number: 22870373.2
(22) Date of filing: 19.09.2022
(51) Int. Cl.: C07K 14/47, A61K 47/64, A61P 35/00

(54) **NOVEL PROTEIN AND PHARMACEUTICAL COMPOSITION COMPRISING SAME FOR PREVENTION OR TREATMENT OF CANCER**

(30) Priority: 17.09.2021 KR 20210125051
(71) Applicant: Cellemedy Co., Ltd, Gyeonggi-do 13605 (KR)
(72) Inventor: LEE, Jee Won, Seoul 06707 (KR); MOON, Ok Jeong, Seoul 02873 (KR)
(74) Representative: dompatent von Kreisler Selting Werner - Partnerschaft von Patent- und Rechtsanwälten mbB
(86) International application number: PCT/KR2022/013967
(87) International publication number: WO 2023/043291

(57) **Abstract**

A ferritin fusion protein according to an embodiment includes a cancer-targeting peptide and a peptide to be cleaved in a cancer cell linked to an outer surface thereof. The ferritin fusion protein may enable efficient cancer-targeting treatment by releasing an anticancer drug specifically in a cancer cell, has excellent anticancer efficacy, and can effectively reduce toxicity to a normal cell, thereby minimizing side effects of anticancer treatment.

## Description

### Technical Field

The present invention relates to a novel protein and a pharmaceutical composition for preventing or treating a cancer comprising the same.

### Background Art

Chemotherapy is a systemic treatment with anti-cancer drugs and is one of the three main cancer treatment methods, along with surgery and radiation therapy. Chemotherapeutic anticancer drugs that are administered to the patients circulate throughout the body through blood vessels, preventing the growth of cancer cells or killing the cancer cells. Traditional anticancer drugs have non-specific cytotoxicity, meaning they attack rapidly proliferating cells, leading to both normal and cancer cell death.

Chemotherapy is still widely used to treat most cancers, and there are many different types of chemotherapy. However, the duration and number of treatments for chemotherapy depends on the type of cancer, the type of anticancer drug, the response to treatment, and the severity of side effects. In general, anticancer drugs affect the DNA or microtubules that are normally present in the cell, so while they have some therapeutic effect on cancer cells, they also have adverse effects on normal cells, and the side effects are usually severe enough to require an average rest period of two to three weeks to allow the damaged normal cells to recover.

In the present invention, in order to maximize the cancer treatment efficacy of existing chemotherapeutic anticancer drugs, a peptide targeting a particular molecule (e.g., epidermal growth factor receptor (EGFR)), which is overexpressed in cancer cells, is genetically introduced on the surface of human heavy chain ferritin (a protein nanoparticle), where the chemotherapeutic anticancer drugs are loaded, thereby enabling the targeted delivery of chemotherapeutic anticancer drugs to cancer. In addition, by linking a peptide that can be cleaved by a specific enzyme that is overexpressed in cancer cells to human heavy chain ferritin, we developed an engineered protein nanoparticle that induces cancer-specific drug release and effectively reduces toxicity to normal cells, minimizing side effects.

### Summary of Invention

### Problems to be Solved by Invention

The objective of the present invention is to combine protein nanoparticles that specifically target cancer cells and have a selective release function of chemotherapeutic anticancer drugs to specifically induce cancer cell death and simultaneously reduce cytotoxicity to normal cells, thereby minimizing side effects caused by conventional chemotherapeutic anticancer drugs.

### Means for Solving Problems

1. A ferritin fusion protein, comprising a cancer-targeting peptide and a peptide to be cleaved in a cancer cell, wherein the cancer-targeting peptide and the peptide to be cleaved in the cancer cell are linked to the outer surface of the ferritin.
2. The ferritin fusion protein of the above 1, wherein the peptide to be cleaved in the cancer cell is a peptide consisting of SEQ ID NO: 1.
3. The ferritin fusion protein of the above 1, further comprising a peptide configured for loading an anticancer drug linked to the peptide to be cleaved in the cancer cell.
4. The ferritin fusion protein of the above 3, wherein the peptide configured for loading the anticancer drug is a peptide comprising an amino acid sequence of DE.
5. The ferritin fusion protein of the above 4, wherein the peptide configured for loading the anticancer drug is an oligopeptide comprising the amino acid sequence of DE repeated 2 to 10 times.
6. The ferritin fusion protein of the above 1, wherein the cancer-targeting peptide has SEQ ID NO: 2.
7. The ferritin fusion protein of the above 1, wherein the ferritin consists of human ferritin heavy chains.
8. The ferritin fusion protein of the above 1, wherein the cancer-targeting peptide and the peptide to be cleaved in the cancer cell are independently linked to N-terminus or C-terminus of the ferritin monomer.
9. The ferritin fusion protein of the above 1, wherein the ferritin is a globular protein consisting of 24 ferritin monomers by self-assembly.
10. A pharmaceutical composition for preventing or treating a cancer, comprising the ferritin fusion protein of any one of the above 1 to 9 and an anticancer drug linked to the peptide to be cleaved in the cancer cell of the ferritin fusion protein.
11. The pharmaceutical composition of the above 10, wherein the anticancer drug is linked to the peptide to be cleaved in the cancer cell via the peptide configured for loading the anticancer drug.
12. The pharmaceutical composition of the above 10, wherein the anticancer drug is a compound having anticancer activity.
13. The pharmaceutical composition of the above 10, wherein the anticancer drug is selected from the group consisting of gemcitabine, mitomycin C, doxorubicin, methotrexate, bleomycin, melphalan, chlorambucil, dacarbazine, cytarabine, fludarabine, pemetrexed, dactinomycin, daunorubicin, idarubicin, cladribine, hydroxycarbamide, thioguanine, bendamustine, temozolomide, azacitidine, clofarabine, decitabine, nelarabine, pralatrexate, epirubicin, eribulin, bexarotene, buserelin, crizotinib, dabrafenib, degarelix, goserelin, ibrutinib, lanreotide, lenvatinib, leuprorelin, mifamurtide, niraparib, pazopanib and raltitrexed.
14. The pharmaceutical composition of the above 10, wherein the cancer is selected from the group consisting of brain cancer, head and neck cancer, bladder cancer, breast cancer, cervical cancer, colon cancer, colorectal cancer, endometrial cancer, esophageal cancer, leukemia, lung cancer, liver cancer, ovarian cancer, pancreatic cancer, prostate cancer, rectal cancer, kidney cancer, gastric cancer, testicular cancer, uterine cancer, vascular tumor, squamous cell carcinoma, adenocarcinoma, small cell carcinoma, melanoma, glioma, neuroblastoma, sarcoma, laryngeal cancer, parotid cancer, biliary tract cancer, thyroid cancer, actinic keratosis, acute lymphocytic leukemia, acute myeloid leukemia, adenoid cystic carcinoma, adenoma, adenoid squamous cell carcinoma, anal canal cancer, anal cancer, anorectal cancer, astrocytoma, ganglion adenocarcinoma, basal cell carcinoma, bile cancer, bone cancer, bone marrow cancer, bronchial cancer, bronchial carcinoma, carcinoid, cholangiocarcinoma, chronic lymphocytic leukemia, chronic myelogenous leukemia, clear cell carcinoma, connective tissue cancer, cystic adenoma, digestive system cancer, duodenal cancer, endocrine system cancer, endoderm sinus tumor, endometrial hyperplasia, endometrial adenocarcinoma, endothelial cell carcinoma, ependymal cell, epithelial cell carcinoma, orbital cancer, focal nodular hyperplasia, gallbladder cancer, palpebral cancer, gastrobasal cancer, gastrinoma, glioblastoma, glucagonoma, heart cancer, hemangioblastoma, hemangioendothelioma, hemangioma, hepatodenoma, liver adenoma, hepatobiliary cancer, hepatocellular carcinoma, Hodgkin's disease, ileal cancer, insulinoma, intraepithelial neoplasia, intraepithelial squamous cell neoplasia, intrahepatic biliary tract cancer, invasive squamous cell carcinoma, jejunum cancer, joint cancer, pelvic cancer, giant cell carcinoma, colon cancer, lymphoma, malignant mesothelial cell tumor, medulloblastoma, medullary epithelioma, meningeal cancer, mesothelial cancer, metastatic carcinoma, oral cancer, mucoepithelial carcinoma, multiple myeloma, muscle cancer, nasal duct cancer, nervous system cancer, non-epithelial skin cancer, non-Hodgkin's lymphoma, soft cell carcinoma, oligodendroglioma, oral cancer, osteosarcoma, papillary serous adenocarcinoma, penile cancer, pharyngeal cancer, pituitary tumor, plasmacytoma, pseudosarcoma, pulmonary blastoma, rectal cancer, renal cell carcinoma, respiratory system cancer, retina blastoma, serous carcinoma, sinus cancer, skin cancer, small cell carcinoma, small intestine cancer, smooth muscle cancer, soft tissue cancer, somatostatin-secreting tumor, spine cancer, squamous cell carcinoma, striatal muscle cancer, submesothelial carcinoma, T cell leukemia, tongue cancer, ureter cancer, urethral cancer, cervical cancer, uterine trunk cancer, vaginal cancer, VIPoma, vulvar cancer, highly differentiated carcinoma, and Wilm's tumor.

### Advantageous effects

A ferritin fusion protein of the present invention may comprise a cancer-targeting peptide and a peptide to be cleaved in a cancer cell linked to the outer surface of the ferritin, and an anticancer drug linked to the peptide to be cleaved in the cancer cell, thereby improving anticancer efficacy, and minimizing side effects.

### Brief Description of the Drawings

FIG. 1 shows vectors for expression of huHF(+), a protein nanoparticle, huHF(-), a protein nanoparticle without cathepsin E cleavage-inducing peptide in huHF(+), and huHF(+/-), a protein nanoparticle without GE11, which is a cancer targeting peptide, in huHF(+).
FIG. 2A shows transmission electron microscope (TEM) images, sizes and models of the protein nanoparticles huHF(+) and anticancer drug conjugates thereof.
FIG. 2B shows transmission electron microscope (TEM) images, sizes and models of the protein nanoparticles huHF(-) and anticancer drug conjugates thereof.
FIG. 2C shows transmission electron microscope (TEM) images, sizes and models of the protein nanoparticles huHF(+/-) and anticancer drug conjugates thereof.
FIG. 3A shows the results of a selective drug release capability verification experiment of protein nanoparticle-chemotherapeutic anticancer drug conjugates using cathepsin E.
FIG. 3B shows the results of a selective drug release capability verification experiment of protein nanoparticle-chemotherapeutic anticancer drug conjugates using lysosomal protease mixture (LPM).
FIG. 4 shows the cytotoxicity verification results of the protein nanoparticle-anticancer drug conjugates, showing the degree of toxicity reduction of the anticancer drug in each cell.
FIG. 5 shows the results of the intracellular selective drug release experiments of protein nanoparticle-anticancer drug conjugates. More specifically FIGS. 5A, 5B and 5C show the results where the anticancer drugs are gemcitabine (GEM), mitomycin C (MMC) and doxorubicin (DOX), respectively.
FIG. 6A shows near-infrared (NIR) image analysis at different times after injection of PBS, huHF(+), huHF(+/-), anticancer drug, and protein nanoparticle-anticancer drug conjugate.
FIG. 6B shows near-infrared (NIR) image analysis in liver, lung, kidney, spleen, heart, and tumor after injection of PBS, huHF(+), huHF(+/-), anticancer drug, and protein nanoparticle-anticancer drug conjugate.
FIG. 6C shows the results of the AST activity assay performed to verify the liver toxicity of the protein nanoparticle-anticancer drug conjugate.
FIG. 7A shows a schematic diagram of an experiment to verify the cancer-specific anticancer effect of the protein nanoparticle-anticancer drug conjugate, and the size of the cancer as a result of the experiment.
FIG. 7B shows optical microscope images of each tissue as a result of toxicity verification with organ tissue analysis.
FIG. 8 is schematic diagrams of activation process of cancer cell-specific anticancer drug of one embodiment of the present invention.

### Mode for Carrying out Invention

Hereinafter, the present invention will be described in detail.

The present invention relates to a ferritin fusion protein in which a cancer-targeting peptide and a peptide to be cleaved in a cancer cell are linked to the outer surface thereof.

The ferritin is not limited in origin and may be derived from various animals including human, or microorganisms.

Human ferritin is composed of heavy chains (21 kDa) and light chains (19 kDa), and the monomers constituting the ferritin have a characteristic of forming a spherical nanoparticle with self-assembly. The ferritin may form a self-assembly of 24 monomers with a spherical three-dimensional structure.

Human ferritin has an outer diameter of about 12 nm and an inner diameter of about 8 nm. The structure of the ferritin monomer is a form in which five α-helix structures, i.e., A helix, B helix, C helix, D helix and E helix, are sequentially linked, and includes an atypical polypeptide, called a loop, connecting the polypeptides of each α-helix structure.

A loop is a region that does not structurally break even if a peptide or small protein antigen is inserted into the ferritin. The peptide-ferritin fusion protein monomer in which the peptide is located in the ferritin monomer may be prepared by fusing the peptide using cloning. The loop connecting helix A and helix B is called the A-B loop, the loop connecting helix B and helix C is called the B-C loop, the loop connecting helix C and helix D is called the C-D loop, and the loop connecting helix D and helix E is called the D-E loop.

Information on the ferritin is publicly available at NCBI (GenBank Accession Nos. NM_000146, NM_002032, etc.).

The ferritin may be composed of ferritin heavy chains, and more specifically, human ferritin heavy chains. Human ferritin heavy chain may be a protein represented by an amino acid sequence of SEQ ID NO: 3 derived from human, and the term "ferritin" may be used interchangeably herein with "human ferritin heavy chain" or "huHF".

The ferritin is made up of several monomers that self-assemble to form an organized structure or pattern. A ferritin fusion protein of the present invention may be a nanoscale protein. For example, 24 ferritin monomers may self-assemble to form the ferritin.

The ferritin fusion protein of the present invention may be spherical. If it is spherical, it may have the diameter of 8 to 50 nm. More specifically, the diameter of the ferritin fusion protein may be 8 nm to 50 nm, 8 nm to 45 nm, 8 nm to 40 nm, 8 nm to 35 nm, 8 nm to 30 nm, 8 nm to 25 nm, 8 nm to 20 nm, or 8 nm to 15 nm.

The ferritin fusion protein of the present invention is obtained by linking a cancer-targeting peptide and a peptide to be cleaved in a cancer cell on its outer surface.

The fusion position is not particularly limited as long as it does not interfere with the self-assembly of the ferritin monomers and may be exposed to the surface of the ferritin monomer. For example, the peptides may be linked between adjacent α-helices, at the N-terminus, at the C-terminus, in the A-B loop, in the B-C loop, in the C-D loop, in the D-E loop, between the N-terminus and the A helix, between the E helix and the C-terminus, or inside the helices, etc.

Although there is an inwardly concave portion in the ferritin monomer, it may protrude outward by the binding of the peptide, causing the peptide to be exposed to the outside.

Specifically, the peptides may be linked to at least one of the sites between adjacent α-helices. Specifically, the peptide also may be linked to the N-terminus or C-terminus of the ferritin monomer. Specifically, the peptide also may be linked between the N-terminus and the A-helix or between the E-helix and the C-terminus of the ferritin monomer. Specifically, the peptide also may be linked to the inside of at least one of the helices of the ferritin monomer.

The term "cancer-targeting peptide" refers to a peptide capable of specifically binding to the cancer cell or cancer tissue both in vivo and in vitro, and it leads the protein of the present invention to the cancer cell to be treated.

The cancer-targeting peptide may be appropriately selected by a person skilled in the art. For example, the cancer-targeting peptide may be a peptide that induces binding to a receptor that is overexpressed in a target cancer cell.

In one embodiment, the cancer-targeting peptide has SEQ ID NO: 2, which can target cancer by binding to an epidermal growth factor receptor (EGFR) that is overexpressed in the cancer cell.

The cancer-targeting peptide may be linked anywhere on the ferritin monomer if it is exposed on the outer surface of the ferritin. The cancer-targeting peptide may be linked to a location that does not interfere with the self-assembly of the ferritin monomers.

The term "peptide to be cleaved in a cancer cell" refers to a peptide comprising a cleavage site that may be cleaved in the cancer cell. The peptide to be cleaved in the cancer cell allows the therapeutic effect to be specific to the cancer cell.

If the peptide can be specifically cleaved in the cancer cell, the cleavage method is not limited, and the peptide may be appropriately selected by the person skilled in the art. For example, the peptide to be cleaved in the cancer cell may be a peptide to be cleaved by an enzyme specifically overexpressed in the cancer cell but is not limited thereto.

In one embodiment, the peptide to be cleaved in the cancer cell may be a peptide to be cleaved in the cancer cell by cathepsin E (CTSE), which is an enzyme overexpressed in the cancer cell.

For example, the peptide to be cleaved in the cancer cell may be a peptide consisting of SEQ ID NO: 1.

In addition, the ferritin fusion protein of the present invention may be further linked with a peptide configured for loading the anticancer drug.

The term "peptide configured for loading an anticancer drug" refers to a peptide utilized to load the anticancer drug to the ferritin fusion protein of the present invention. The peptide may be linked to the peptide to be cleaved in the cancer cell.

The bond between the peptide and the anticancer drug may be by an ionic bond, a covalent bond, a coordination bond, a hydrogen bond, an electrostatic attraction, a van der Waals force, a hydrophobic bond, or the like, but is not limited thereto. Preferably, the bond between the peptide and the anticancer drug may be the covalent bond.

Once the person skilled in the art selects the anticancer drug suitable for a treatment, the person may appropriately select the peptide configured for loading the anticancer drug and link it to the ferritin. For example, the peptide configured for loading the anticancer drug may be a peptide having a functional group capable of loading the selected anticancer drug.

Specifically, for example, if the anticancer drug having an amino group is selected for the treatment, the peptide configured for loading the anticancer drug may have a plurality of carboxy groups to load the anticancer drug. The anticancer drug may be bound to the peptide by an EDC-NHS binding reaction (EDC(1-ethyl-3-[3-dimethylaminopropyl] carbodiimide)-NHS(N-hydroxysuccinimide)).

In addition, the peptide having the functional group capable of binding to the anticancer drug, the peptide modified to have the functional group, or the anticancer drug modified to have the functional group may be used to load the anticancer drug on the peptide.

The peptide configured for loading the anticancer drug may comprise an amino acid sequence, DE.

The peptide configured for loading the anticancer drug may comprise the amino acid sequence, DE, repeated a plurality of times to load the plurality of anticancer drugs. For example, the amino acid sequence, DE, may be repeated 2 to 10 times, 2 to 8 times, 2 to 6 times, 2 to 5 times, or 2 to 4 times. In an aspect, the peptide configured for loading the plurality of anticancer drugs may have the amino acid sequence, DE, repeated 2 to 4 times.

The term "anticancer drug" refers to a therapeutic agent including a chemotherapeutic agent used to treat or prevent a hyperproliferative disease, such as a cancer.

The anticancer drug may be directly linked to the peptide to be cleaved in the cancer cell or may be indirectly linked to the peptide to be cleaved in the cancer cell via the peptide configured for loading the anticancer drug. As described above, the anticancer drug may be loaded by ionic bonding, covalent bonding, or the like.

The anticancer drug may be a compound that has anticancer activity.

For example, the anticancer drug may be selected from the group consisting of gemcitabine, mitomycin C, doxorubicin, methotrexate, bleomycin, melphalan, chlorambucil, dacarbazine, cytarabine, fludarabine, pemetrexed, dactinomycin, daunorubicin, idarubicin, cladribine, hydroxycarbamide, thioguanine, bendamustine, temozolomide, azacitidine, clofarabine, decitabine, nelarabine, pralatrexate, epirubicin, eribulin, bexarotene, buserelin, crizotinib, dabrafenib, degarelix, goserelin, ibrutinib, lanreotide, lenvatinib, leuprorelin, mifamurtide, niraparib, pazopanib and raltitrexed, but is not limited thereto.

The protein of the present invention may further comprise a linker.

The linker may include, for example, glycine. Specifically, the linker may be G3SG3TG3SG3, but is not limited thereto.

For example, the peptide to be cleaved in the cancer cell may be linked to the ferritin via the linker. In such a case, the peptide may be more easily cleaved in the cancer cell by being exposed to the outside, and the binding efficiency between the anticancer drug and the peptide configured for loading the anticancer drug may be increased.

Furthermore, in the present invention, the cancer-targeting peptide and the peptide to be cleaved in the cancer cell may be independently linked to N-terminus or C-terminus of the ferritin. For example, if the cancer-targeting peptide is introduced at the N-terminus of the ferritin monomer, the peptide to be cleaved in the cancer cell may be introduced at the C-terminus of the ferritin. For example, if the peptide to be cleaved in the cancer cell is introduced at the N-terminus of the ferritin, the cancer-targeting peptide may be introduced at the C-terminus of the ferritin. For example, if the linker or the like is further included, the cancer-targeting peptide may be introduced at the N-terminus of the ferritin, and the linker or the like may be further included between the C-terminus of the ferritin and the peptide to be cleaved in the cancer cell. Alternatively, the linker or the like may be further included between the N-terminus of the ferritin and the peptide to be cleaved in the cancer cell, and the cancer-targeting peptide may be introduced at the C-terminus of the ferritin.

In the present invention, the ferritin monomers may self-assemble to form an organized structure or pattern. The ferritin fusion protein of the present invention may be a nanoscale particle. For example, 24 of the ferritin monomers may self-assemble to form a spherical ferritin protein.

The present invention also provides a pharmaceutical composition for preventing or treating the cancer comprising the ferritin fusion protein.

The pharmaceutical composition of the present invention may comprise the ferritin fusion protein and the anticancer drug which is linked to the peptide to be cleaved in the cancer cell. All of the matters described above with respect to the ferritin fusion protein are applicable to the ferritin fusion protein as an active ingredient in the pharmaceutical composition of the present invention. In addition, the term "anticancer drug" refers to the therapeutic agent used to treat or prevent the hyperproliferative disease, such as the cancer, as described above.

The pharmaceutical composition of the present invention may comprise a pharmaceutically acceptable carrier. As used herein, the term "pharmaceutically acceptable carrier" refers to a carrier or diluent that does not significantly irritate the organism and does not inhibit the biological activity and the property of the administered component. The pharmaceutically acceptable carrier in the present invention may be one of saline, sterile water, Ringer's solution, buffered saline, dextrose solution, maltodextrin solution, glycerol, ethanol and others, or may be a mixture of one or more thereof. Further, other conventional additives, such as antioxidants, buffers and bacteriostatic agents, may be added as needed to formulate an injection suitable for injection into a tissue or organ. It may also be formulated as an isotonic sterile solution, or in some cases, a dry preparation (particularly a lyophilized preparation) that can be an injectable solution by adding sterile water or physiological saline. In addition, a target organ-specific antibody or other ligands may be combined with the carrier so as to act specifically on the target organ.

The composition of the present invention may further comprise fillers, excipients, disintegrants, binders, or lubricants. The composition of the present invention may also be formulated by methods known in the art to provide a rapid, sustained or delayed release of the active ingredient after administration to a mammal.

In one embodiment, the pharmaceutical composition may be an injectable formulation and may be administered intravenously, but is not limited thereto.

As used herein, the term "effective amount" refers to an amount sufficient to delay or prevent the onset or progression of a specific disease to be treated, or to alleviate a condition to be treated.

In the present invention, the composition may be administered in a pharmaceutically effective amount. It will be apparent to those skilled in the art that an adequate total daily dosage of the pharmaceutical composition may be determined by the practitioner within the scope of sound medical judgment.

The pharmaceutically effective amount of the composition of the present invention may vary depending on a variety of factors including: the type and extent of a biological response to be achieved; the age, weight, general health condition, sex and diet of a patient; the time and route of administration; the rate of discharge and composition ratio of the composition; the duration of treatment; whether any other drug is used together or concurrently with the composition; and other factors well known in the pharmaceutical field.

As used herein, the term "administration" or "administering" refers to introducing the composition of the present invention to the patient by any suitable methods. The composition of the present invention may be administered by various routes, for example, oral or parenteral routes, as long as it may reach the target tissue. The composition of the present invention may be administered intraperitoneally, intravenously, intramuscularly, subcutaneously, intradermally, orally, topically, intranasally, intrapulmonary, or rectally, but the route of administration is not limited thereto.

In the present invention, the pharmaceutical composition may be packaged in a form as directed by a government agency in charge of the manufacture, use and sale of drugs, and may be accompanied by an instruction related thereto. The instruction may indicate the approval of a private organization for the form of the composition or for administration to humans or animals, or may indicate the approval of the US Food and Drug Administration for the prescription of the drug, for example.

The anticancer drug that may be included in the present invention are as described above.

The protein of the present invention comprising the anticancer drug allows the targeted therapy as it releases the anticancer drug specifically in the cancer cell, i.e., it kills the cancer cell with low cytotoxicity to the normal cell.

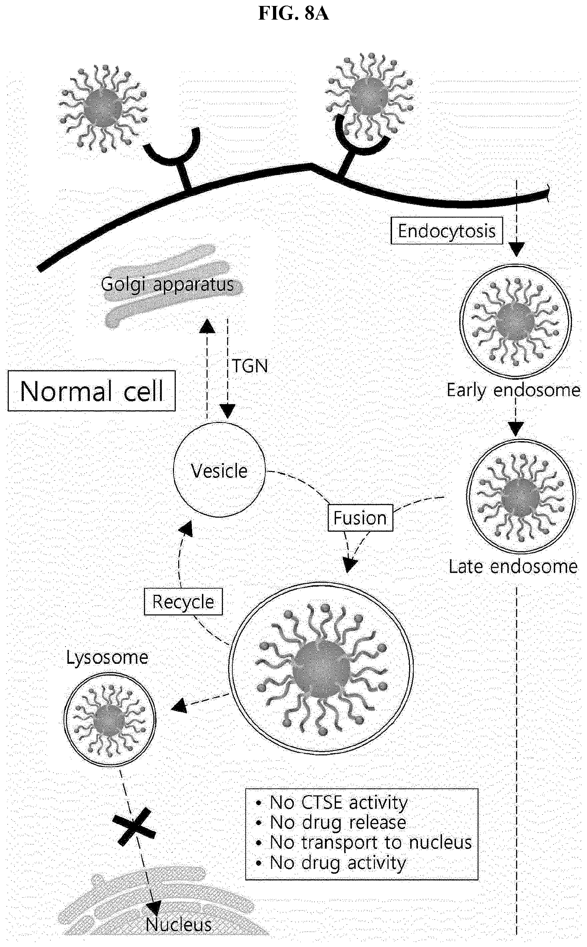

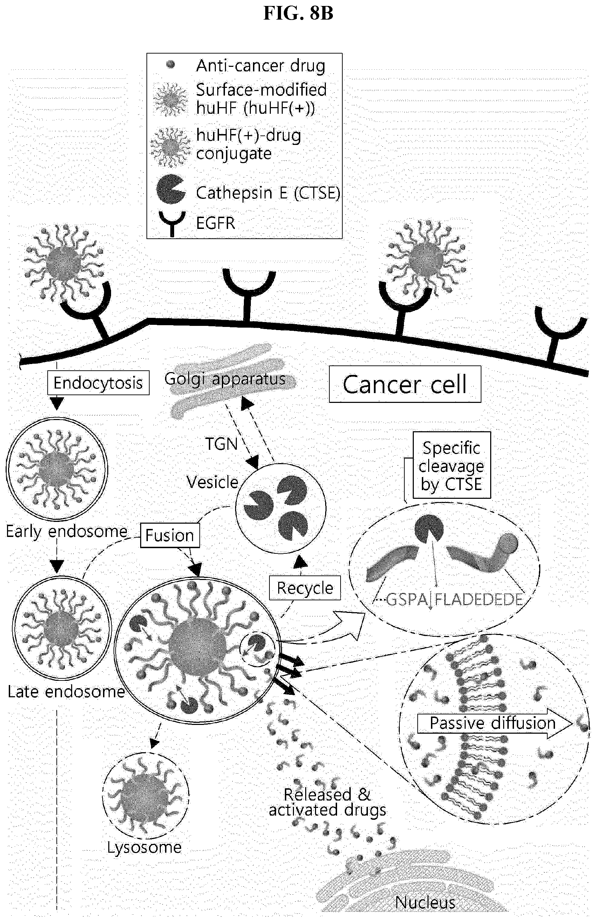

Specifically, as shown in FIG. 8, the anticancer activity in the cancer cell and normal cell administered with the composition of the present invention is significantly different. The cancer-targeting peptide, the peptide to be cleaved in the cancer cell, and the peptide configured for loading the anticancer drug may be linked to the surface of the human ferritin heavy chain (huHF). For example, the "cancer-targeting peptide", "peptide to be cleaved in the cancer cell" and "peptide configured for loading the anticancer drug" linked to the huHF surface may be a peptide targeting epidermal growth factor receptors (EGFRs) overexpressed in the cancer cell, a peptide to be cleaved by cathepsin E (CTSE) which is an enzyme overexpressed in the cancer cell (for example, a peptide consisting of SEQ ID NO: 1), and an oligopeptide which has the amino acid sequence, DE, repeated three times and is linked to the peptide to be cleaved in the cancer cell, respectively. Hereinafter, the term "huHF(+)-anticancer drug conjugate" refers to the ferritin protein linked to the peptides and the anticancer drug.

The huHF(+)-anticancer drug conjugate may be transported to the cancer cell by the cancer-targeting peptide targeting epidermal growth factor receptors (EGFRs) overexpressed in the cancer cell, and be introduced to the cancer cell by endocytosis. Then, the peptide may be cleaved by cathepsin E (CTSE), an enzyme overexpressed in the cancer cell. The anticancer drug linked the peptide to be cleaved in the cancer cell may be cleave and released from the huHF(+)-anticancer drug conjugate and activated. The released anticancer drug may then be finally transported to the nucleus to exhibit anticancer activity, such as chromosomal DNA damage and/or DNA replication interference.

In contrast, huHF(+)-anticancer drug is rarely brought into the normal cell by endocytosis. Furthermore, the peptide is not cleaved by cathepsin E (CTSE) activity in the normal cell. Consequently, the anticancer drug is not released and remains inactive, and it is not transported to the nucleus and thus does not show anticancer activity.

Therefore, the pharmaceutical composition of the present invention may be used for the treatment or prevention of the cancer.

The cancer may be, for example, selected from the group consisting of brain cancer, head and neck cancer, bladder cancer, breast cancer, cervical cancer, colon cancer, colorectal cancer, endometrial cancer, esophageal cancer, leukemia, lung cancer, liver cancer, ovarian cancer, pancreatic cancer, prostate cancer, rectal cancer, kidney cancer, gastric cancer, testicular cancer, uterine cancer, vascular tumor, squamous cell carcinoma, adenocarcinoma, small cell carcinoma, melanoma, glioma, neuroblastoma, sarcoma, laryngeal cancer, parotid cancer, biliary tract cancer, thyroid cancer, actinic keratosis, acute lymphocytic leukemia, acute myeloid leukemia, adenoid cystic carcinoma, adenoma, adenoid squamous cell carcinoma, anal canal cancer, anal cancer, anorectal cancer, astrocytoma, ganglion adenocarcinoma, basal cell carcinoma, bile cancer, bone cancer, bone marrow cancer, bronchial cancer, bronchial carcinoma, carcinoid, cholangiocarcinoma, chronic lymphocytic leukemia, chronic myelogenous leukemia, clear cell carcinoma, connective tissue cancer, cystic adenoma, digestive system cancer, duodenal cancer, endocrine system cancer, endoderm sinus tumor, endometrial hyperplasia, endometrial adenocarcinoma, endothelial cell carcinoma, ependymal cell, epithelial cell carcinoma, orbital cancer, focal nodular hyperplasia, gallbladder cancer, palpebral cancer, gastrobasal cancer, gastrinoma, glioblastoma, glucagonoma, heart cancer, hemangioblastoma, hemangioendothelioma, hemangioma, hepatodenoma, liver adenoma, hepatobiliary cancer, hepatocellular carcinoma, Hodgkin's disease, ileal cancer, insulinoma, intraepithelial neoplasia, intraepithelial squamous cell neoplasia, intrahepatic biliary tract cancer, invasive squamous cell carcinoma, jejunum cancer, joint cancer, pelvic cancer, giant cell carcinoma, colon cancer, lymphoma, malignant mesothelial cell tumor, medulloblastoma, medullary epithelioma, meningeal cancer, mesothelial cancer, metastatic carcinoma, oral cancer, mucoepithelial carcinoma, multiple myeloma, muscle cancer, nasal duct cancer, nervous system cancer, non-epithelial skin cancer, non-Hodgkin's lymphoma, soft cell carcinoma, oligodendroglioma, oral cancer, osteosarcoma, papillary serous adenocarcinoma, penile cancer, pharyngeal cancer, pituitary tumor, plasmacytoma, pseudosarcoma, pulmonary blastoma, rectal cancer, renal cell carcinoma, respiratory system cancer, retina blastoma, serous carcinoma, sinus cancer, skin cancer, small cell carcinoma, small intestine cancer, smooth muscle cancer, soft tissue cancer, somatostatin-secreting tumor, spine cancer, squamous cell carcinoma, striatal muscle cancer, submesothelial carcinoma, T cell leukemia, tongue cancer, ureter cancer, urethral cancer, cervical cancer, uterine trunk cancer, vaginal cancer, VIPoma, vulvar cancer, highly differentiated carcinoma, and Wilm's tumor, but is not limited thereto.

Hereinafter, the present invention will be described in detail with reference to the following examples. The examples are provided only for better understanding of the present invention and should not be construed as limiting the scope of the present invention.

### Example

### 1. Construction of expression vectors for the biosynthesis of human ferritin-derived nanoparticles

According to the vector schematic diagram shown in Table 1 below, huHF(+), and two controls, huHF(-) and huHF(+/-) were prepared using PCR, where huHF(+) is a human ferritin-derived protein in which GE11, cathepsin E cleavage-inducing peptide and chemotherapeutic anticancer drug binding-inducing peptide were linked and expressed, huHF(-) is huHF(+) without cathepsin E cleavage-inducing peptide, and huHF(+/-) is huHF(+) without GE11. All of the prepared plasmid expression vectors were purified on agarose gels, and then sequences of each thereof were identified by total DNA sequencing.

The PCR products were sequentially inserted into the pT7-7 expression vector to construct expression vectors capable of expressing each protein nanoparticle.

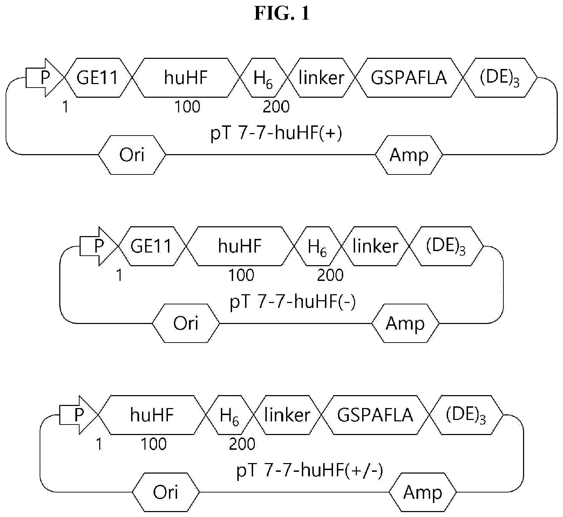

Vectors for expressing each of the protein nanoparticles are pT7-huHF(+), pT7-huHF(-) and pT7-huHF(+/-) (FIG. 1).

mm

**[Table 1] Expression vector constructs for each nanoparticle [113]**

| Protein nanoparticle | Expression vector |
|---|---|
| huHF(+) | *NH₂-Nde* I -GE11-huHF-*Xho* I -H₆-linker(G3SG3TG3SG3)- GSPAFLA-(DE)₃-*Hind* III-COOH |
| huHF(-) | *NH₂-Nde* I -GE11-huHF-*Xho* I -H₆-linker(G3SG3TG3SG3)-(DE)₃-*Hind* III-*COOH* |
| huHF(+/-) | *NH₂-Nde* I -huHF-*Xho* I -H₆-linker(G3SG3TG3SG3)- GSPAFLA-(DE)₃-*Hind* III-COOH |

### 2. Biosynthesis and Purification of Candidate Protein Nanoparticles

*E. coli* strain BL21(DE3)[F-ompThsdSB(rB-mB-)] was transformed with each of the expression vectors prepared as described above, and ampicillin-resistant transformants were selected. The transformed *E. coli* was cultured in a flask (250 mL Erlenmeyer flask, 37 °C, 150 rpm) containing 50 mL of Luria-Bertani (LB) medium (containing 100 mg L⁻¹ ampicillin). When the medium turbidity (O.D600) reached about 0.4 to 0.5, IPTG (Isopropyl-β-D-thiogalactopyranosid) (1.0 mM) was added to induce the expression of the recombinant gene. After culturing at 20 °C for 16 to 18 h, the cultured *E. coli* was centrifuged at 4,500 rpm for 10 min to obtain a cell precipitate. The obtained cell precipitate was then suspended in 5 mL of lysis solution (10 mM Tris-HCl buffer, pH 7.5, 10 mM EDTA) and lysed using an ultrasonicator (Branson Ultrasonics Corp., Danbury, CT, USA). The lysate was centrifuged at 13,000 rpm for 10 minutes to separate the supernatant and insoluble aggregates. The separated supernatant was subjected to Ni²⁺-NTA affinity chromatography using the binding of nickel to histidine linked to the recombinant protein, and then the recombinant protein was concentrated and a buffer was exchanged to obtain purified recombinant protein. Each step is described in detail below.

### 1) Ni²⁺-NTA affinity chromatography

To purify the recombinant protein, *E. coli* cultured according to the aforementioned method were collected, and the cell pellet was resuspended in 5 mL lysis solution (50 mM NaH₂PO₄, 300 mM NaCl, 10 mM imidazole, 0.2 mM CuSO₄, pH 8.0), followed by lysis using an ultrasonicator. The lysate was centrifuged at 13,000 rpm for 10 min to isolate the supernatant, and each recombinant protein was separated using a Ni²⁺-NTA column (Quiagen, Hilden, Germany) (washing buffer: 50 mM NaH₂PO₄, 300 mM NaCl, 10 mM imidazole, pH 8.0 / elution buffer: 50 mM NaH₂PO₄, 300 mM NaCl, 250 mM imidazole, pH 8.0).

### 2) Concentration and buffer exchange

2 mL of the recombinant protein eluted through Ni²⁺-NTA affinity chromatography was placed in ultracentrifugal filter (Amicon Ultra 100K, Millipore, Billerica, MA) and centrifuged at 5,000 rpm until 1 mL of solution remained on the column. The buffer was then exchanged with PBS buffer (137 mM NaCl, 2.7 mM KCl, 10 mM Na₂HPO₄, 1.8 mM KH₂PO₄, pH 7.4).

### 3. Formation of binding between prepared protein nanoparticles and chemotherapeutic anticancer drugs and analysis of the structure thereof

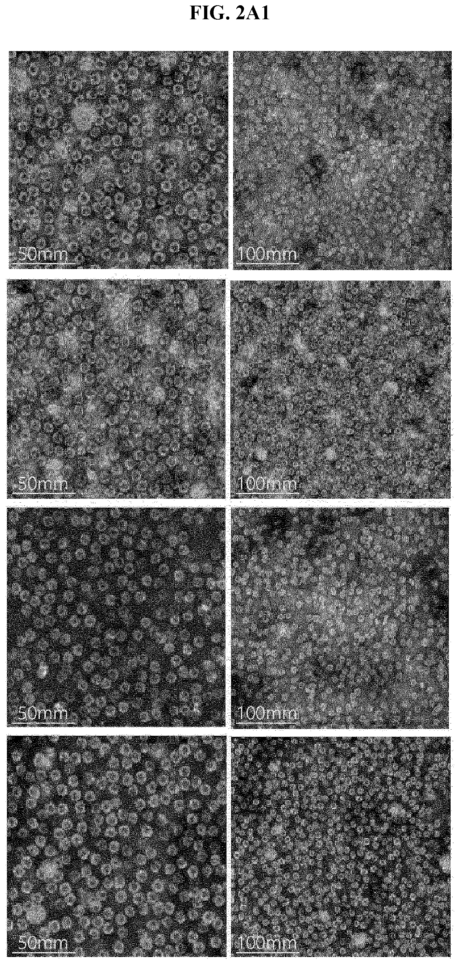

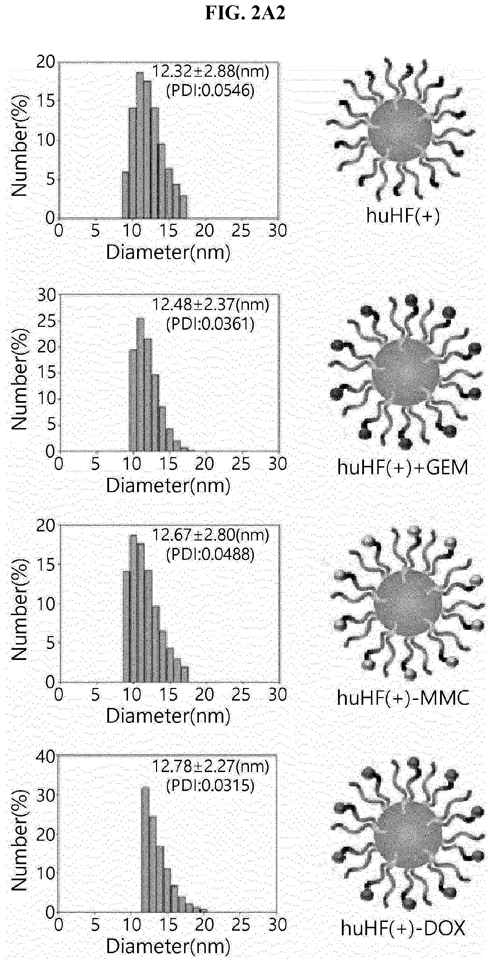

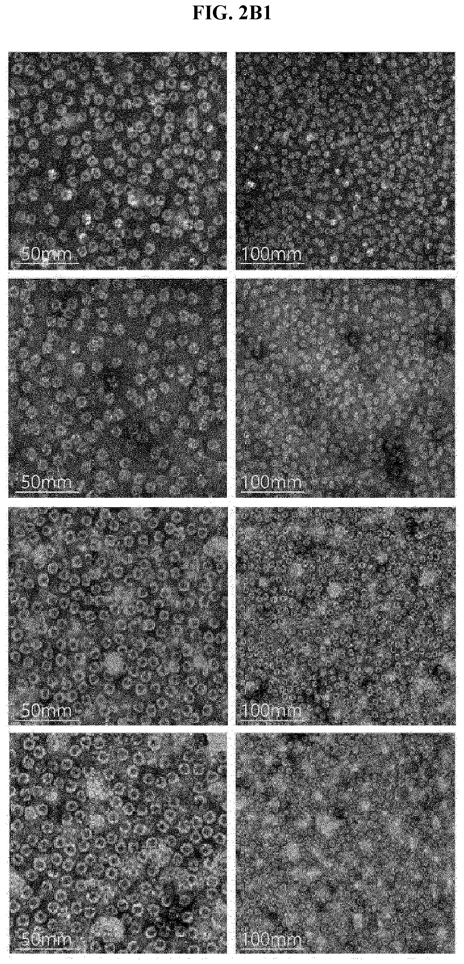

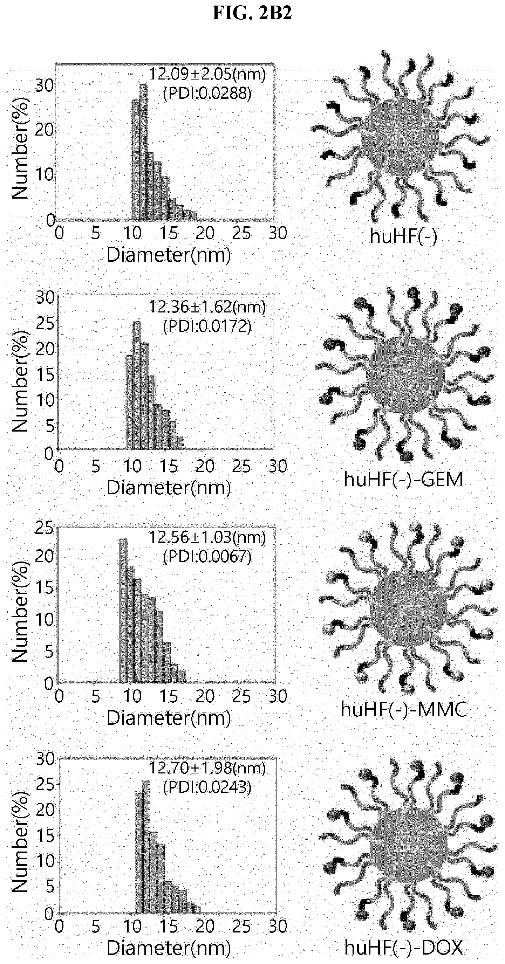

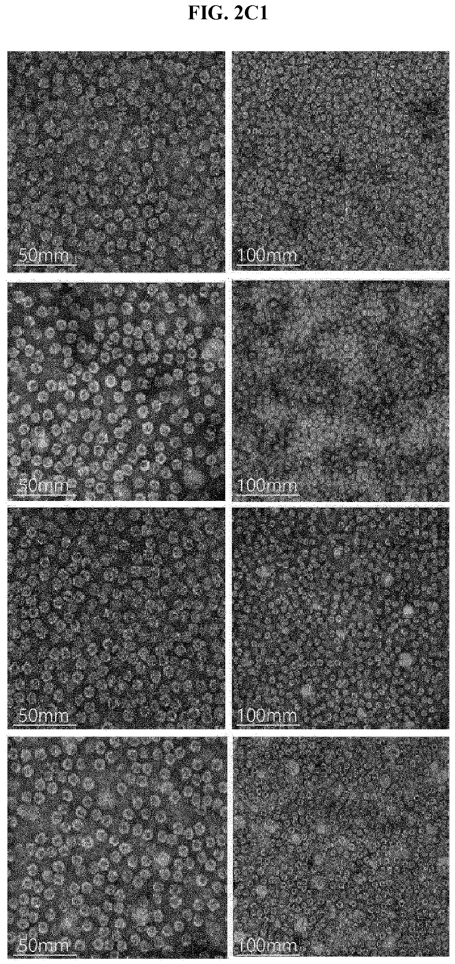

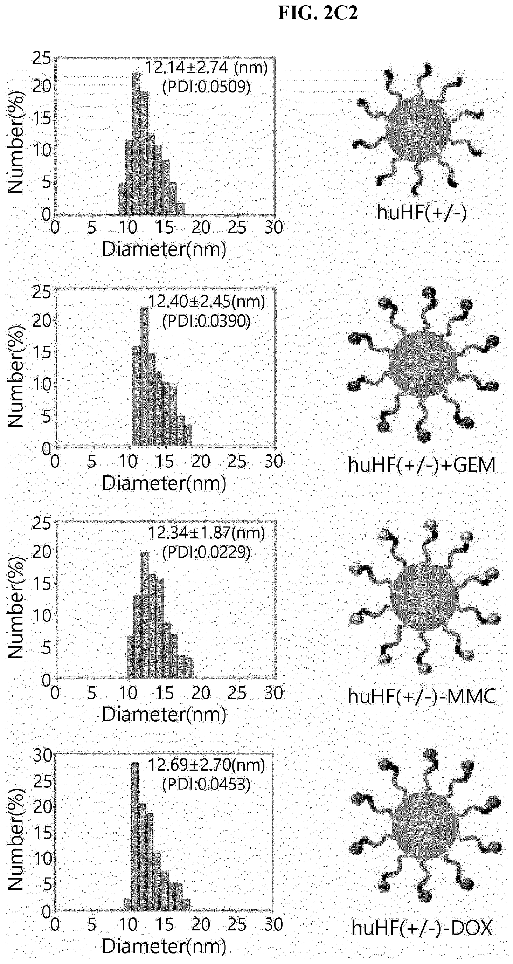

After the above steps, EDC (1-ethyl-3-[3-dimethylaminopropyl]carbodiimide)-NHS (N-hydroxysuccinimide) reaction was carried out to form a bond between the purified recombinant protein nanoparticles and chemotherapeutic anticancer drugs containing amine groups such as gemcitabine (GEM), mitomycin C (MMC), and doxorubicin (DOX). An EDC-NHS reaction buffer was prepared by adding 0.4 mg of EDC and 1.1 mg of Sulfo-NHS to 1 ml of a 2-[morpholino]ethanesulfonic acid (0.1 M, pH 6.0) buffer. Fifteen minutes after adding 22 µL of EDC-NHS reaction buffer per 1 mL of recombinant protein nanoparticles at room temperature, chemotherapeutic anticancer drugs GEM, MMC and DOX were reacted at room temperature for 2 hours with molar ratio (molar concentration of chemotherapeutic anticancer drug : molar concentration of recombinant protein nanoparticle) of 288:1, 720:1 and 144:1, respectively. Then, in order to remove the chemotherapeutic anticancer drug remaining unbound, the reactant was placed in ultracentrifugal filter (Amicon Ultra 100K, Millipore, Billerica, MA) to exchange the reaction buffer with PBS buffer (137 mM NaCl, 2.7 mM KCl, 10 mM Na₂HPO₄, 1.8 mM KH₂PO₄, pH 7.4). The purified recombinant protein nanoparticles and the recombinant protein nanoparticle-chemotherapeutic anticancer drug conjugates prepared as described above were photographed using transmission electron microscopy (TEM) for structural analysis. Electron microscope grids containing air-dried samples were incubated with 2% (w/v) aqueous uranyl acetate solution for 1 h at room temperature to obtain stained images of protein nanoparticles. They were observed using a Tecnai 20 (FEI, Hillsboro, Oregon, U.S.A.) electron microscope operating at 200 kV to confirm that both protein nanoparticles and protein nanoparticle-anticancer drug conjugates form spherical nanoparticles. Furthermore, DLS (dynamic light scattering) analysis showed that huHF(+) forms a spherical nanoparticle having a size of 12.32 ± 2.88 nm, huHF(+)-GEM forms a spherical nanoparticle having a size of 12.48 ± 2.37 nm, huHF(+)-MMC forms a spherical nanoparticle having a size of 12.67 ± 2.80 nm, huHF(+)-DOX forms a spherical nanoparticle having a size of 12.78 ± 2.27 nm, huHF(-) forms a spherical nanoparticle having a size of 12.09 ± 2.05 nm, huHF(-)-GEM forms a spherical nanoparticle having a size of 12.36 ± 1.62 nm, huHF(-)-MMC forms a spherical nanoparticle having a size of 12.56 ± 1.03 nm, huHF(-)-DOX forms a spherical nanoparticle having a size of 12.70 ± 1.98 nm, huHF(+/-) forms a spherical nanoparticle having a size of 12.14 ± 2.74 nm, huHF(+/-)-GEM forms a spherical nanoparticle having a size of 12.40 ± 2.45 nm, huHF(+/-)-MMC forms a spherical nanoparticle having a size of 12.34 ± 1.87 nm, and huHF(+/-)-DOX forms a spherical nanoparticle having a size of 12.69 ± 2.70 nm (FIGS. 2A, 2B and 2C).

### 4. Verification of selective drug release ability of protein nanoparticle-chemotherapeutic anticancer drug conjugates

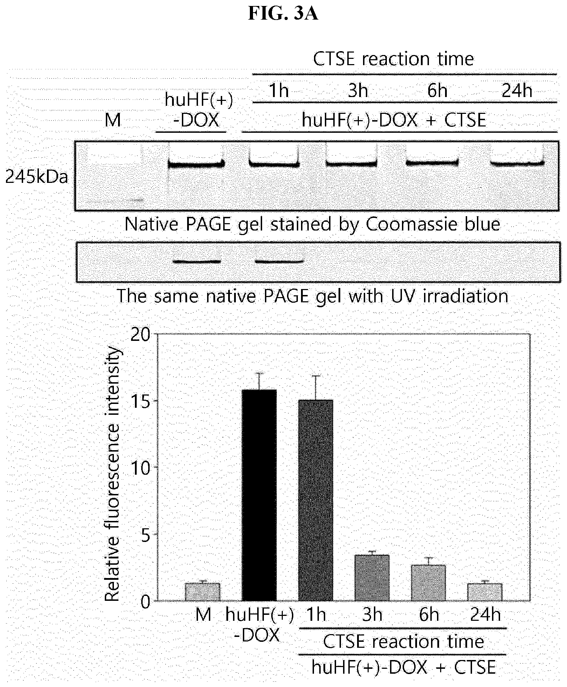

To verify whether the recombinant protein nanoparticle-chemotherapeutic anticancer drug conjugate could be cleaved by cathepsin E to selectively release the drug, experiments were conducted using doxorubicin, which is naturally fluorescent. The recombinant protein and doxorubicin were conjugated as described above, and the protein nanoparticle-anticancer drug conjugate was reacted with cathepsin E enzyme (10 U/ml) at 40 °C, pH 5.0, the optimal conditions for cathepsin E enzyme, over time. The reacted protein nanoparticle-anticancer drug conjugate was then subjected to native-PAGE using a 12% Tris-glycine precast gel (Invitrogen, California, U.S.A.). The gel was stained with Coomassie blue staining solution to quantify protein nanoparticles. From 3 hours after staining, it was visually observed that the portion to which the anticancer drug was bound in the conjugate was effectively cleaved by cathepsin E. The intensity of doxorubicin was graphed by measuring its UV absorbance (FIG. 3A).

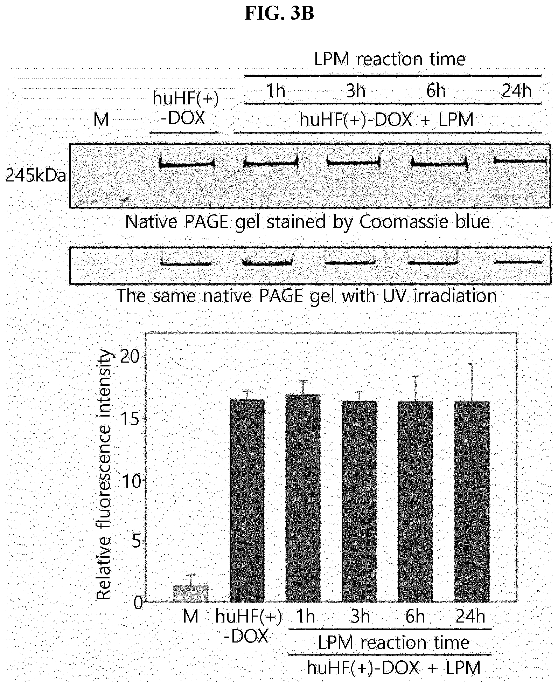

As a result of conducting the experiment in the same manner as described above except that LPM (lysosomal protease mixture), a mixture of various enzymes cathepsins B, D, L, H and PrCP (prolylcarboxypeptidase), which are known to be distributed in lysosomes, was used instead of Cathepsin E, it was observed that the peptide was not specifically cleaved thereby the drug was not selectively released (FIG. 3B).

### 5. Verification of non-cytotoxicity of protein nanoparticle-chemotherapeutic anticancer drug conjugate

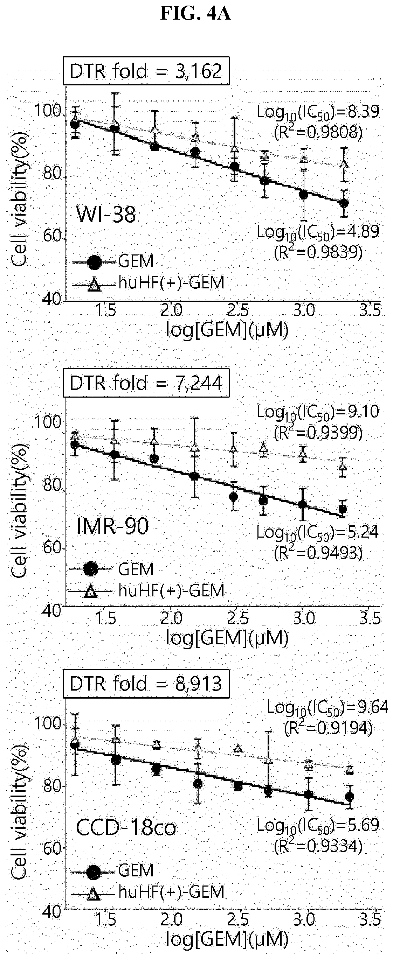

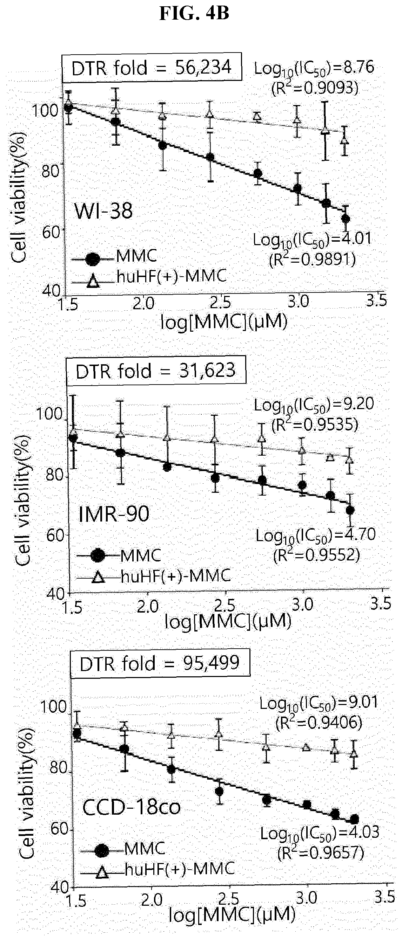

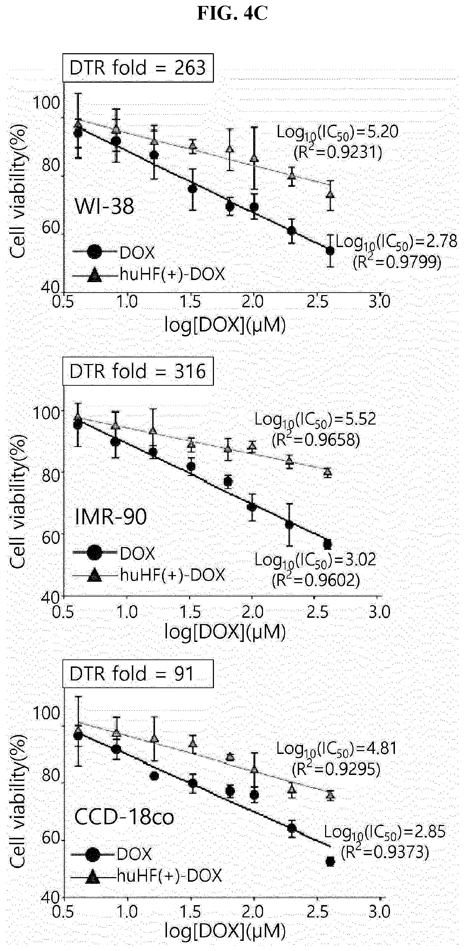

To verify that the protein nanoparticle-anticancer drug conjugate is not toxic in normal cells due to selective drug release as described above, CCK (cell counting kit) assay was performed in various normal cells. Normal lung cells WI-38 and IMR-90, and normal colon cells CCD-18co were grown in 96-well microplate with 1×10⁴ cells/well for 36 hours, and the cytotoxicity when only anticancer drug was added and the cytotoxicity when protein nanoparticle-anticancer drug conjugate was added were compared. By gradually increasing the concentration of the anticancer drug or protein nanoparticle-anticancer drug conjugate from 500 µM to 2000 µM, the IC₅₀, the concentration of the drug at which the cellular activity reached 50%, was obtained, and it was found that the cytotoxicity was effectively reduced when the anticancer drug was conjugated with protein nanoparticles. The drug toxicity reduction (DTR) of the anticancer drugs was calculated, and the DTR of GEM, MMC and DOX in WI-38 cell was 3162, 56234 and 263, respectively; the DTR of GEM, MMC and DOX in IMR-90 cell was 7244, 31623 and 316, respectively; and the DTR of GEM, MMC and DOX in CCD-18co cell was 8913, 95499 and 91, respectively (FIG. 4).

### 6. Observation of selective drug release of protein nanoparticle-chemotherapeutic anticancer drug conjugate in cancer cells and normal cells

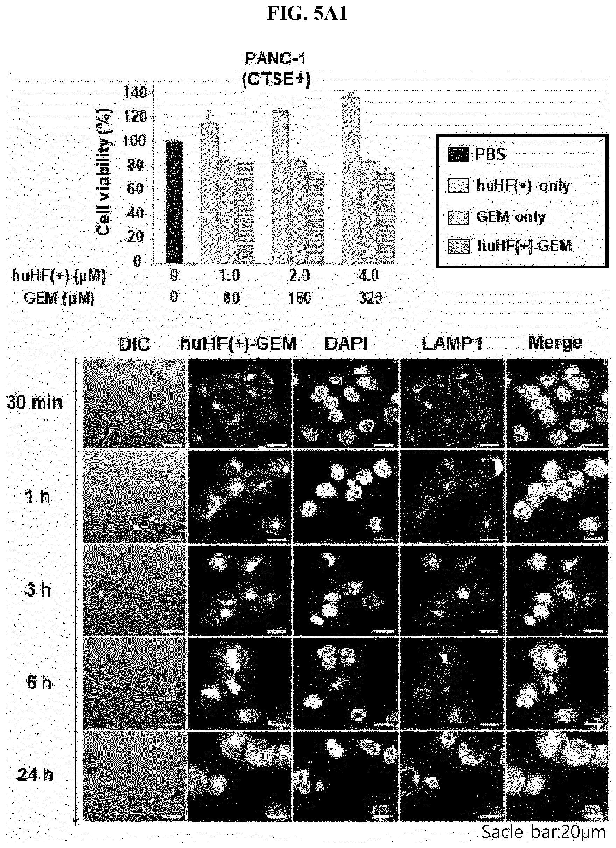

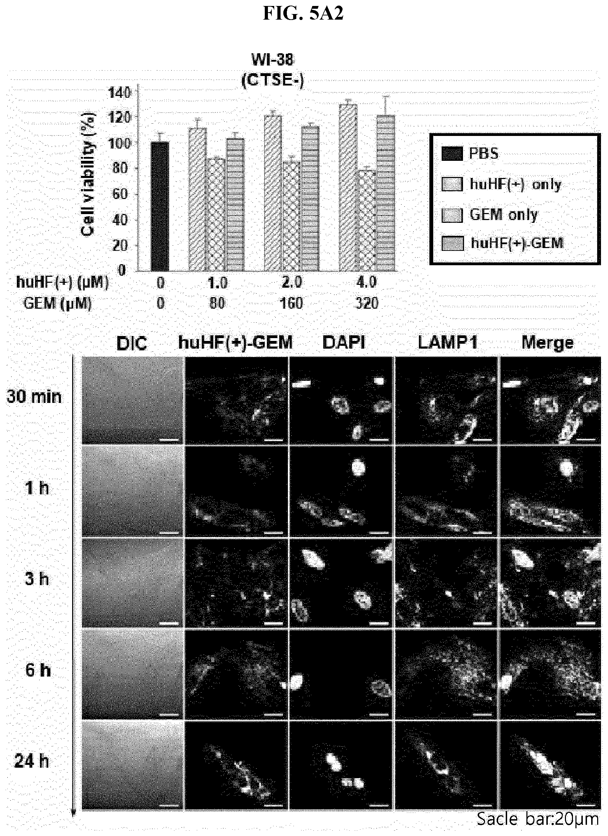

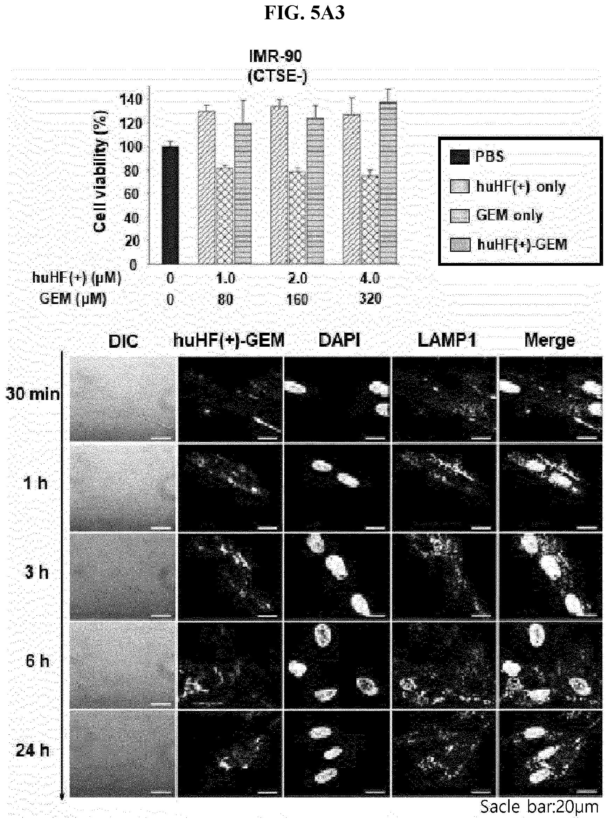

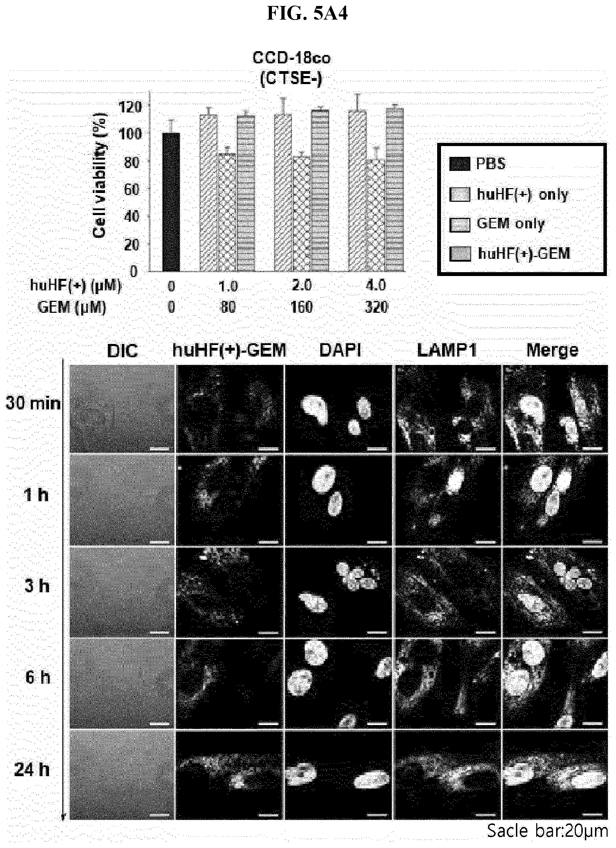

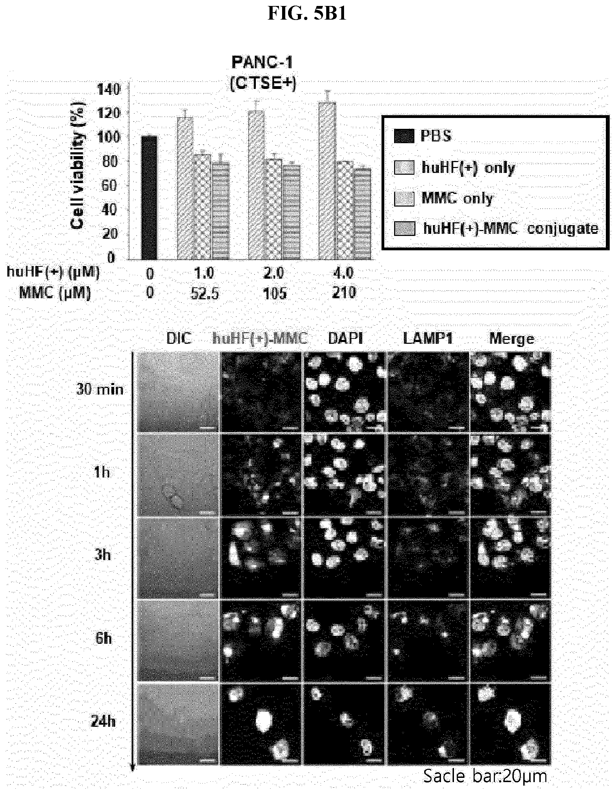

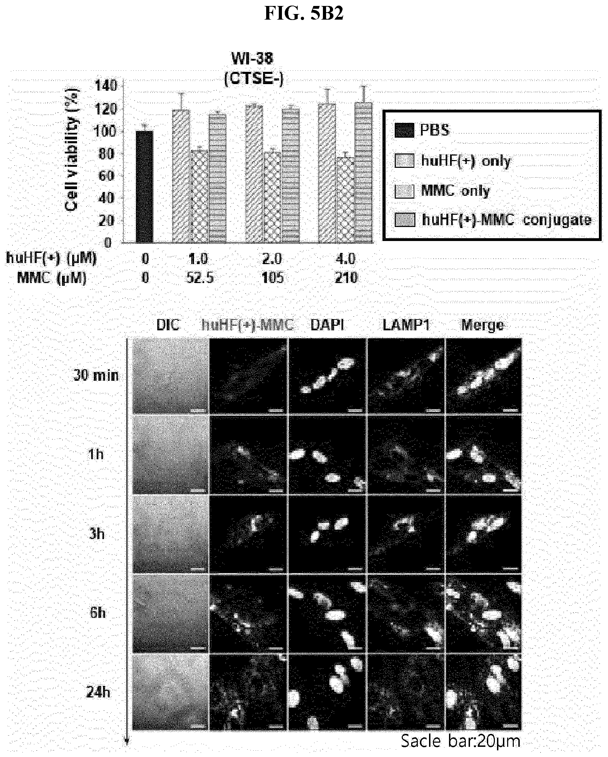

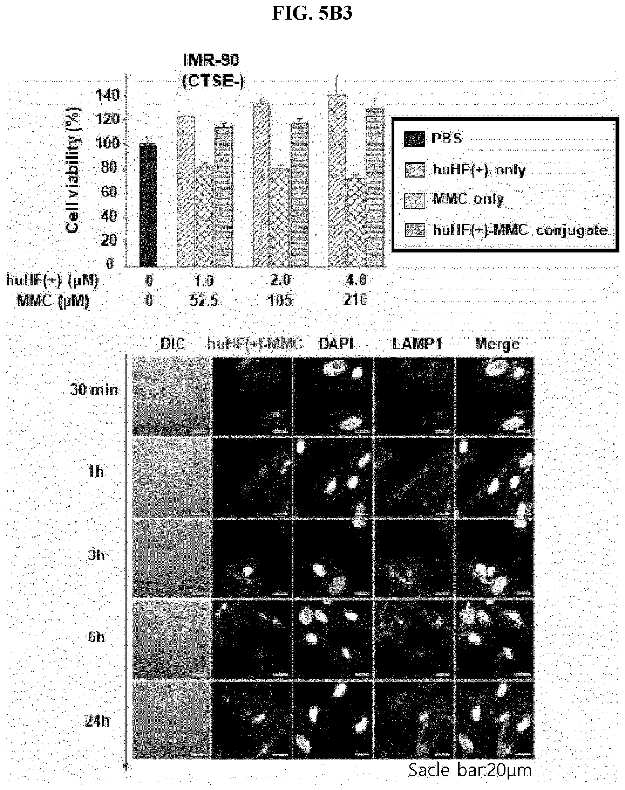

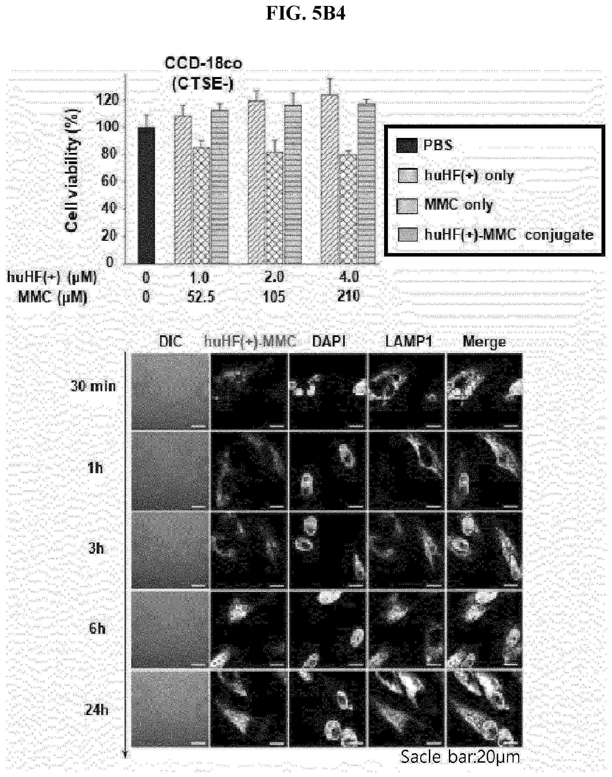

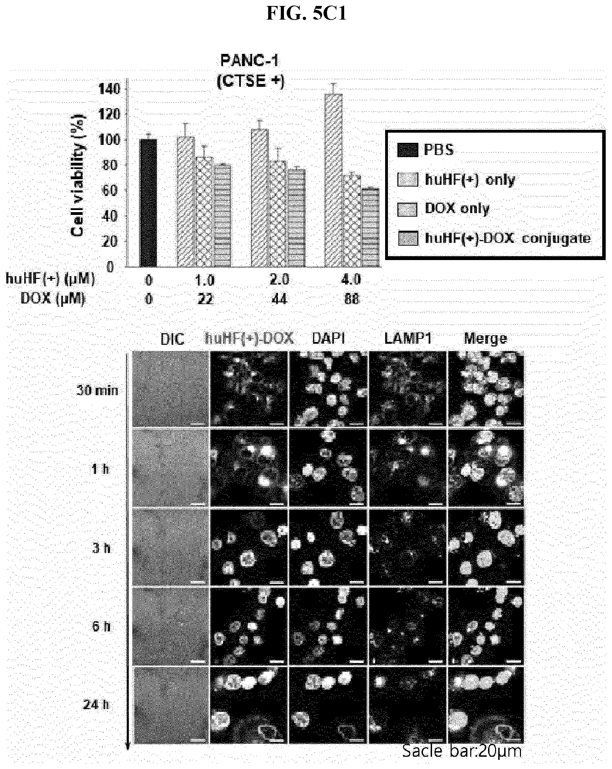

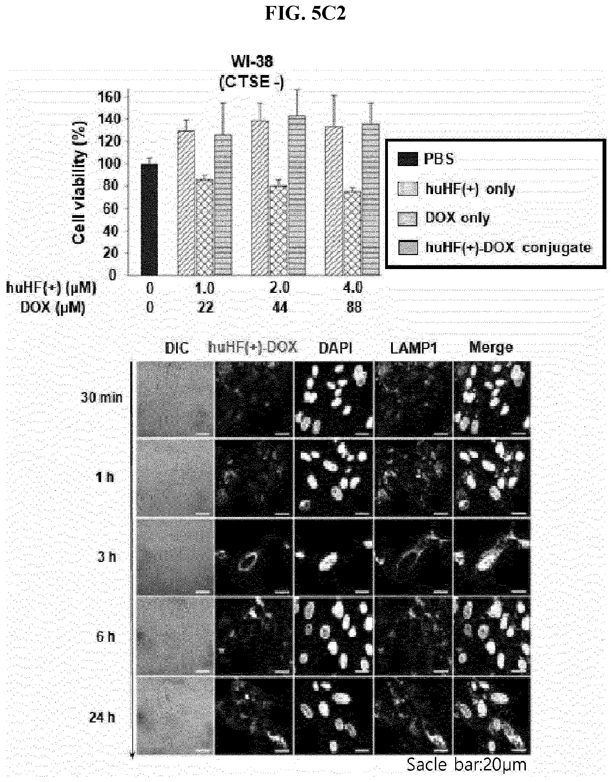

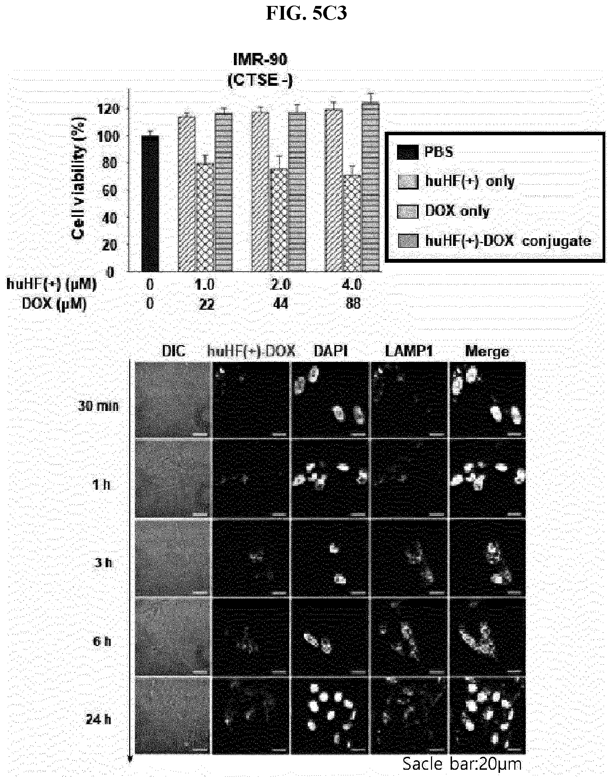

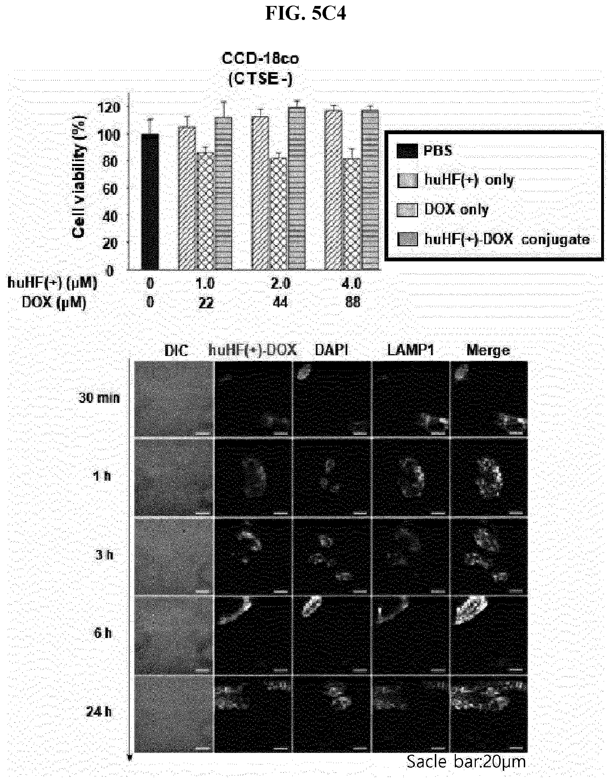

To verify that the protein nanoparticle-anticancer drug conjugate is effective for selective drug delivery to a cancer cell, huHF(+)-DOX (FIG. 5C), huHF(+)-GEM (FIG. 5A) and huHF(+)-MMC (FIG. 5B) were treated with cancer cells, PANC-1, and normal cells, WI-38, IMR-90 and CCD-18co to observe the drug release over time, wherein huHF(+)-DOX was used as it is because doxorubicin is a natural fluorophore, while gemcitabine and mitomycin C were observed by attaching a fluorescent material. The results verified that the drugs were released specifically in the cancer cells and entered the nucleus of the cells, while the drugs were not released in all normal cells. Furthermore, when the CCK assay was performed in the same manner as in Example 5, it was confirmed that the introduction of the protein nanoparticle conjugate increased the cytotoxicity to cancer cells and did not cause cytotoxicity to normal cells compared to the addition of the anticancer drug alone.

### 7. NIR image analysis of protein nanoparticle-chemotherapeutic anticancer drug conjugates and verification of their non-toxicity to the liver

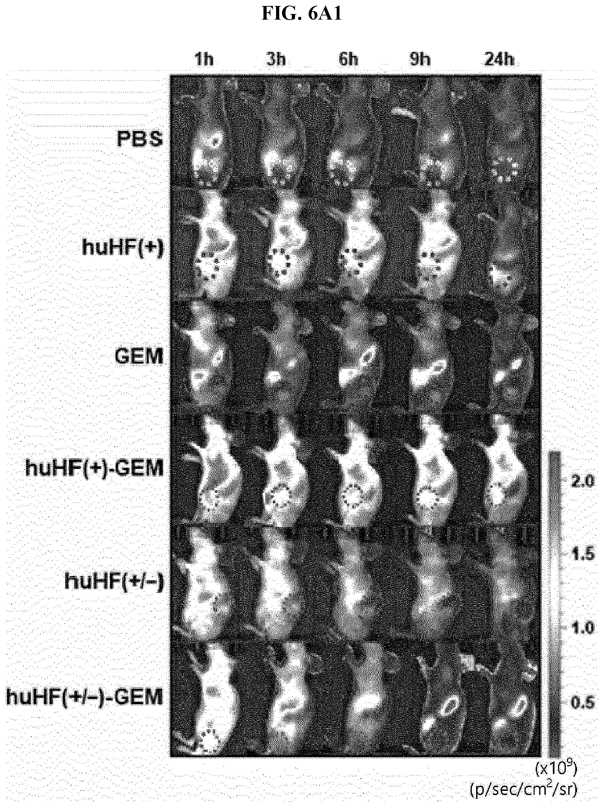

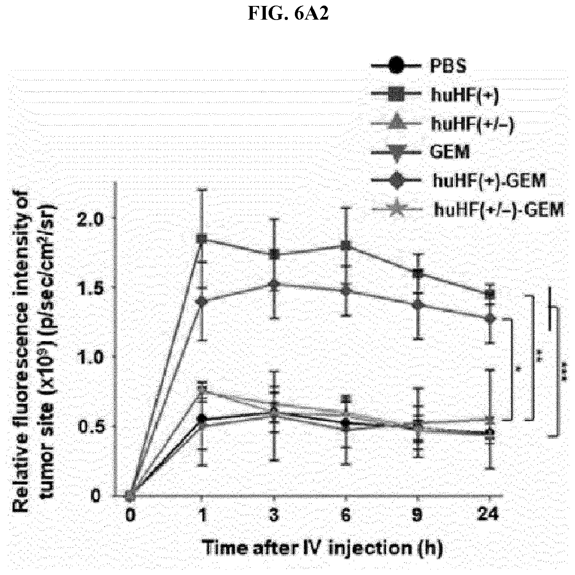

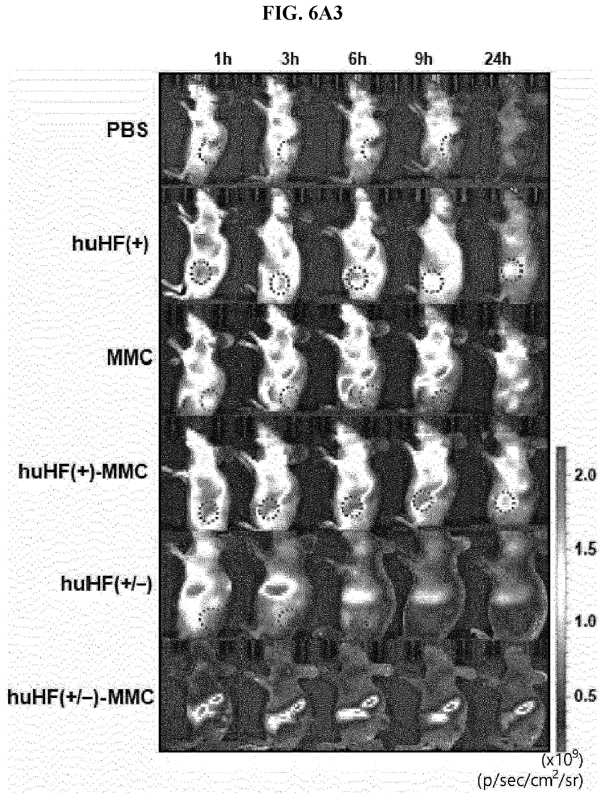

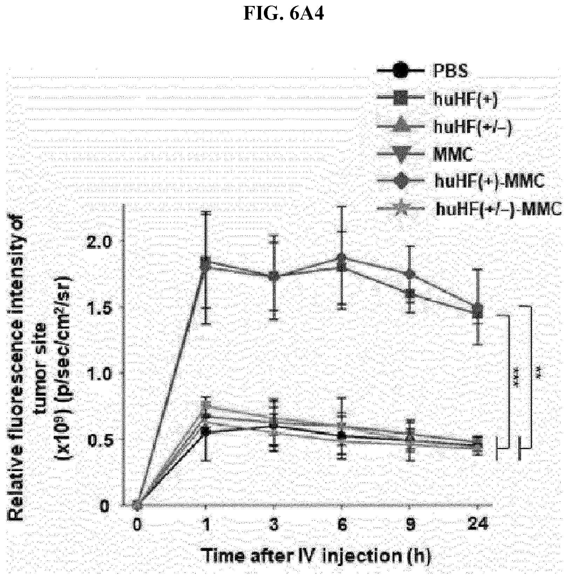

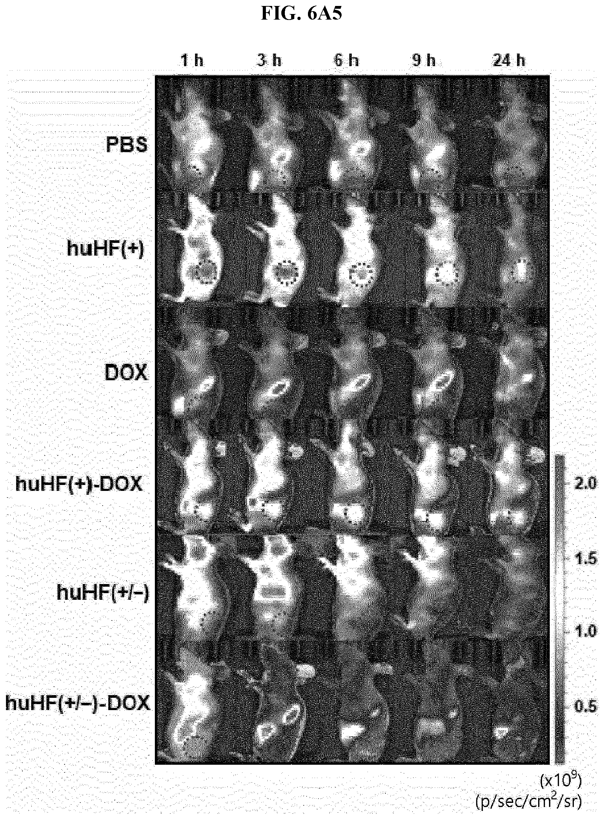

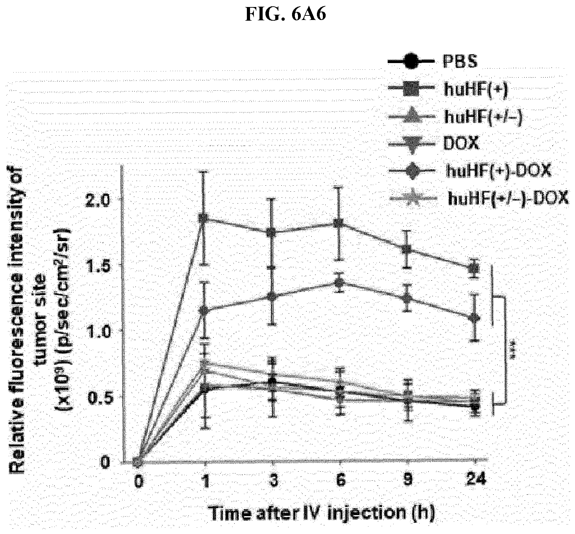

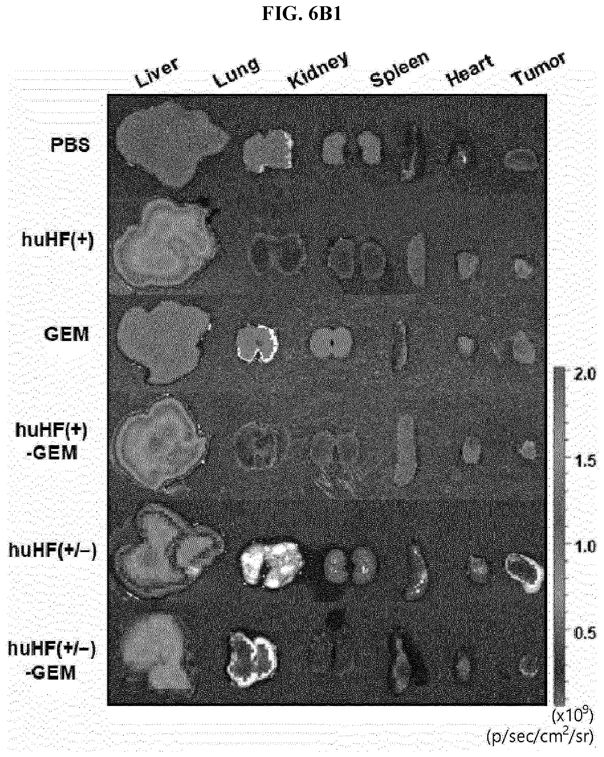

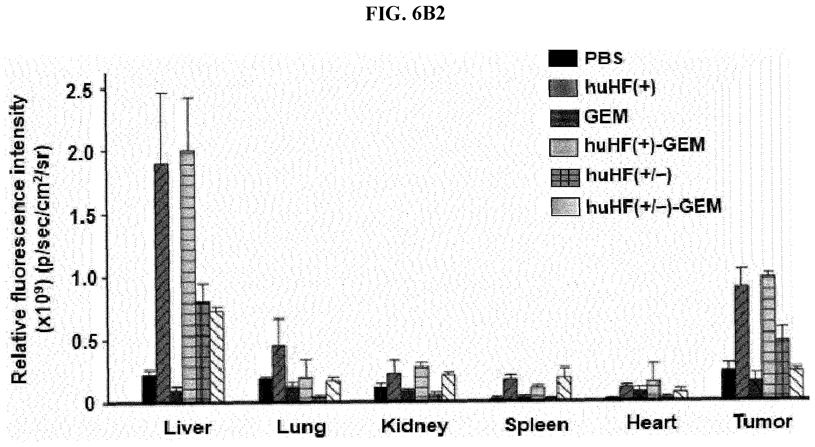

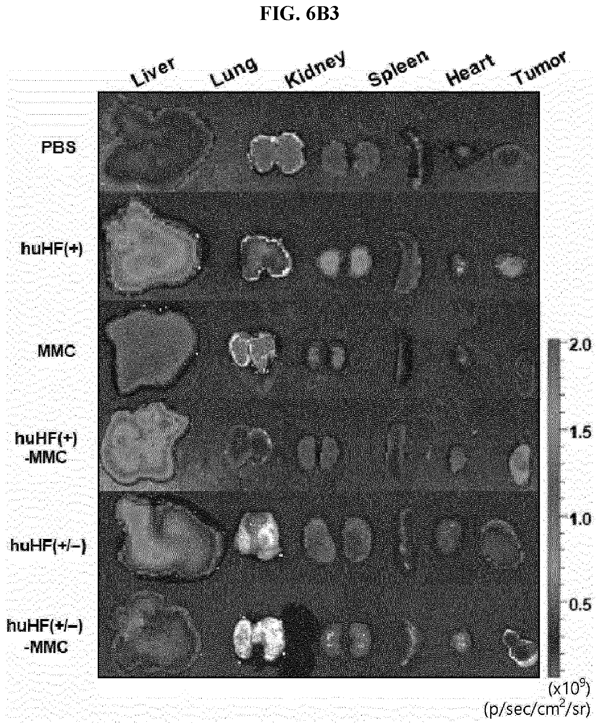

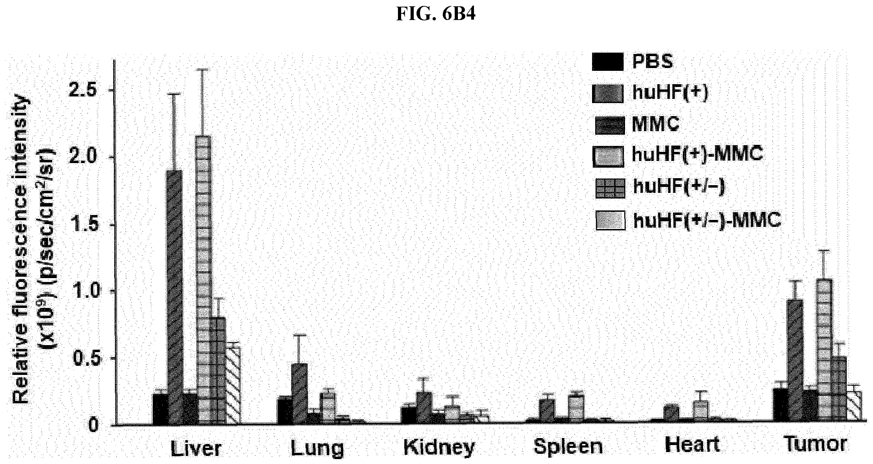

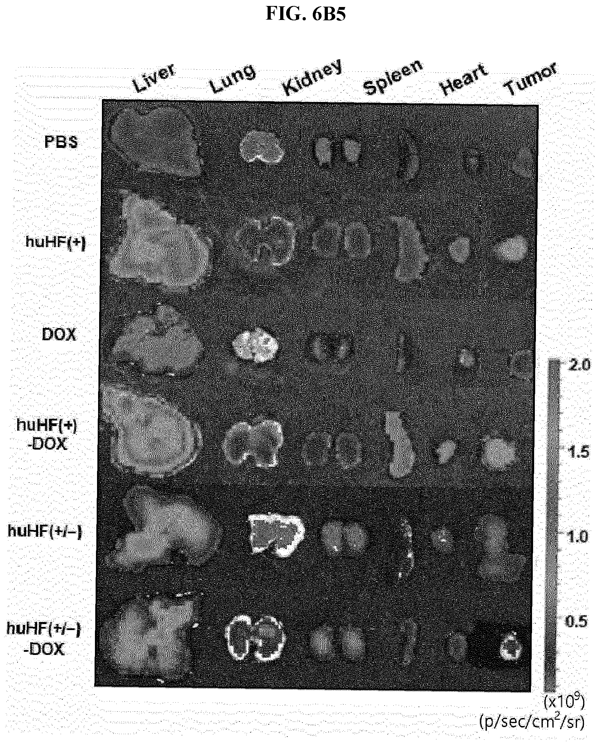

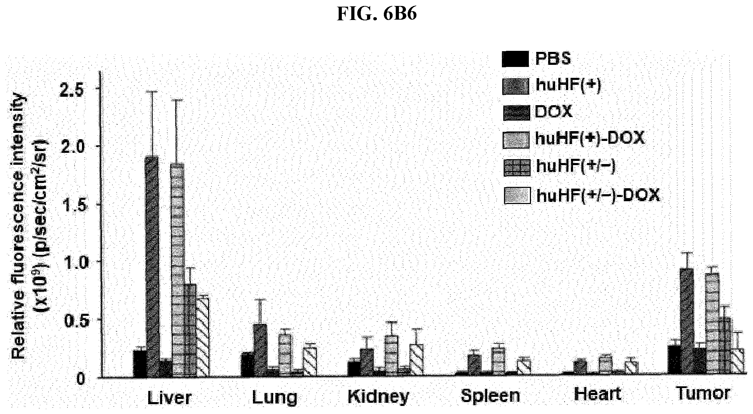

In the same manner as in Example 6, DOX having natural fluorescence properties, and GEM and MMC having fluorescent materials attached thereto were bound to the surface of the nanoparticles, respectively, and then the fluorescence thereof was adjusted to be the same, and injected into a 5-week-old nude mouse to prove that each of the huHF(+)-GEM, huHF(+)-MMC and huHF(+)-DOX conjugates could target cancer, and it was observed that the cancer targeting efficiency was effectively increased by remaining in the cancer for 24 hours. On the contrary, it was observed that huHF(+/-)-GEM, huHF(+/-)-MMC and huHF(+/-)-DOX in which GE11, a cancer targeting peptide, were removed had significantly reduced cancer targeting efficiency (FIGS. 6A and 6B).

Protein nanoparticle-chemotherapeutic anticancer drug conjugates not only target cancer, but also may transport to the liver. AST (aspartate aminotransferase) is mainly present in the liver, and in healthy people, the blood AST concentration is low. When liver toxicity occurs, the liver does not function properly and AST is released into the bloodstream in large amounts.

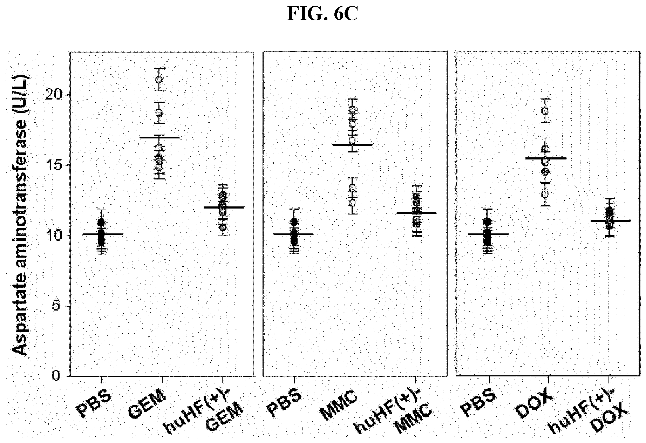

The protein nanoparticle-chemotherapeutic anticancer drug conjugate, the anticancer drug and PBS buffer alone were administered to nude mice, respectively, and 24 hours later, blood was collected from nude mice, and AST activity assay was performed to measure hepatotoxicity. As a result, it was observed that the anticancer drugs bound to the protein nanoparticles significantly decreased the activity of AST compared to the anticancer drug alone (FIG. 6C). This verified that the protein nanoparticle-anticancer drug conjugate can be transported to the liver in large amounts but do not cause toxicity.

### 8. Cancer specific anticancer effect of protein nanoparticle-chemotherapeutic anticancer drug conjugate and non-toxicity verification through long-term tissue analysis

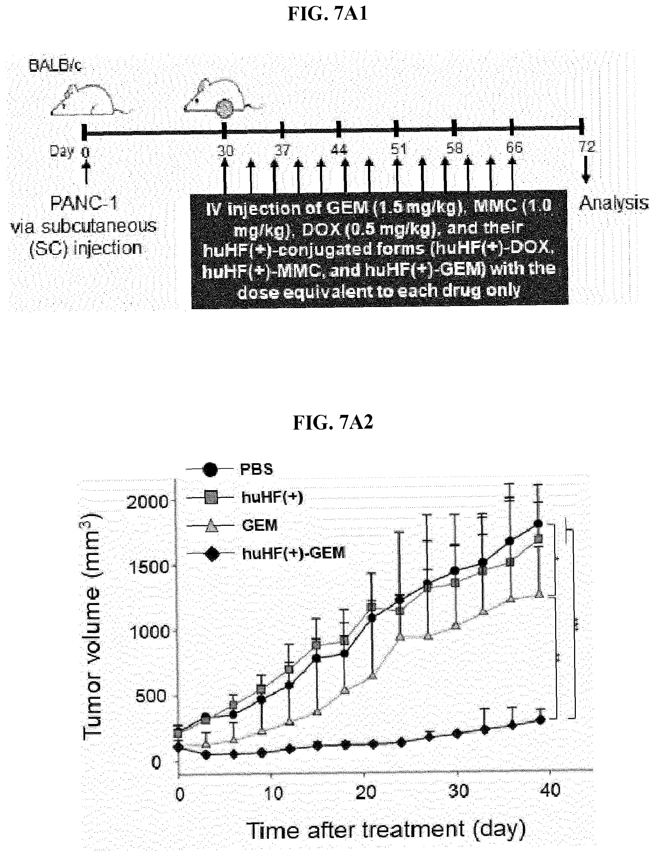

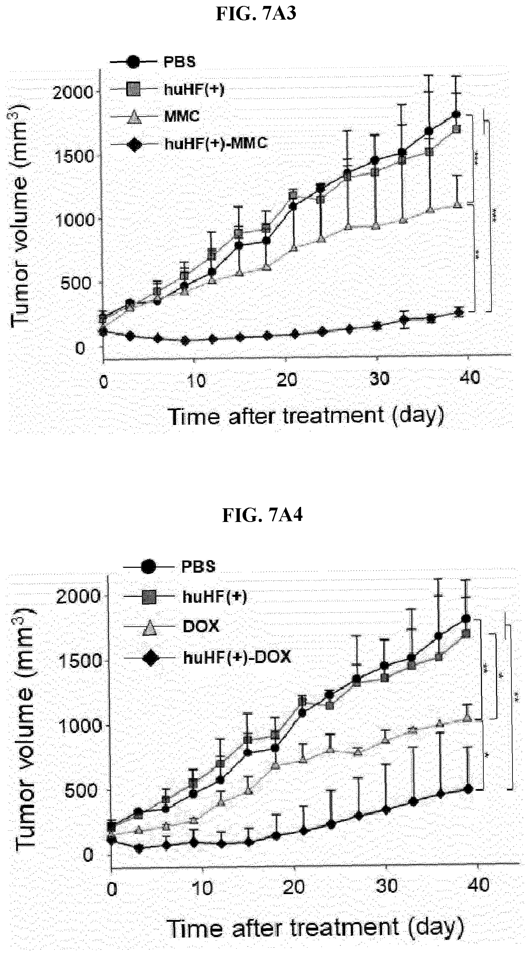

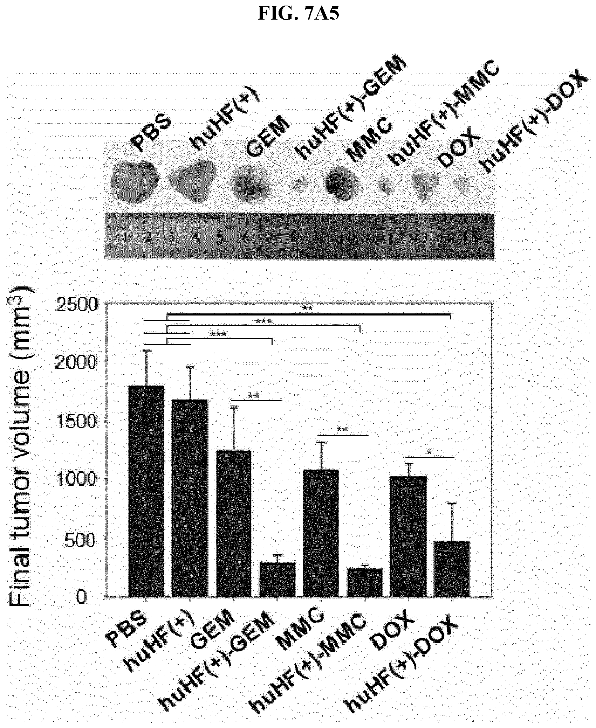

Example 7 demonstrates that chemotherapeutic anticancer drugs conjugated to protein nanoparticles such as huHF(+)-GEM, huHF(+)-MMC and huHF(+)-DOX effectively target cancer and persist in the cancer for a long time. Therefore, we expected that in vivo injection of these protein nanoparticle-chemotherapeutic anticancer drug conjugates would show excellent anti-cancer effects since they specifically target cancer, so we conducted in vivo experiments to verify the extent of cancer size reduction in nude mice after intravenous injection of the conjugates at three-day intervals compared to the injection of buffer, protein nanoparticles, and anticancer drugs alone. The concentration of protein and the anticancer drug in the injections were the same. The volume of the cancer was measured over 42 days, and cancer growth was effectively inhibited when the anticancer drug was bound to protein nanoparticles and injected, compared to the anticancer drug alone. In addition, after 42 days, the cancers of each experimental group were extracted and their sizes were measured, and as a result, when the anticancer drugs were delivered by binding to the protein nanoparticles, all of them showed excellent anticancer effects (FIG. 7A).

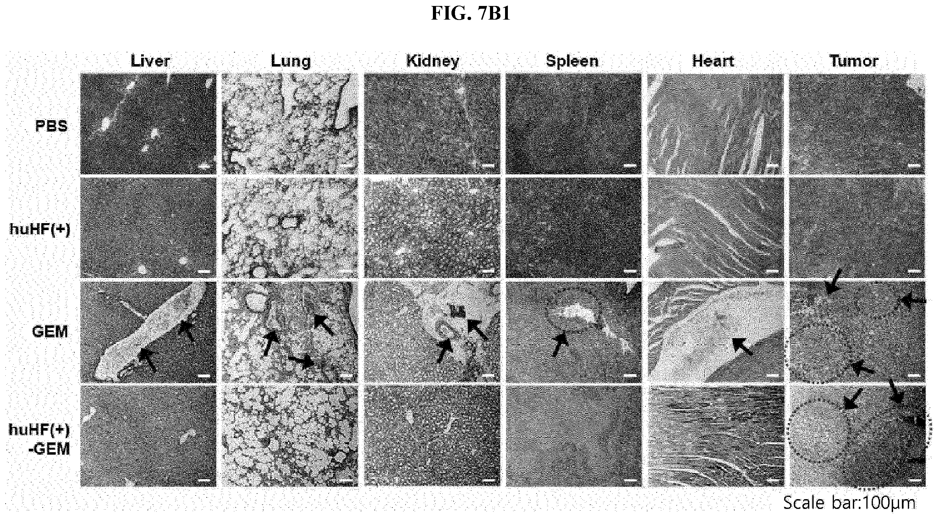

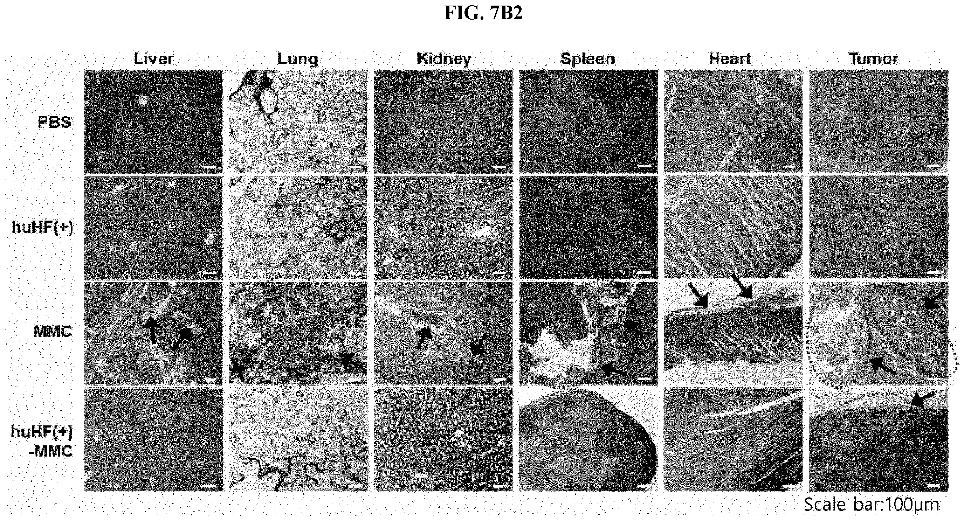

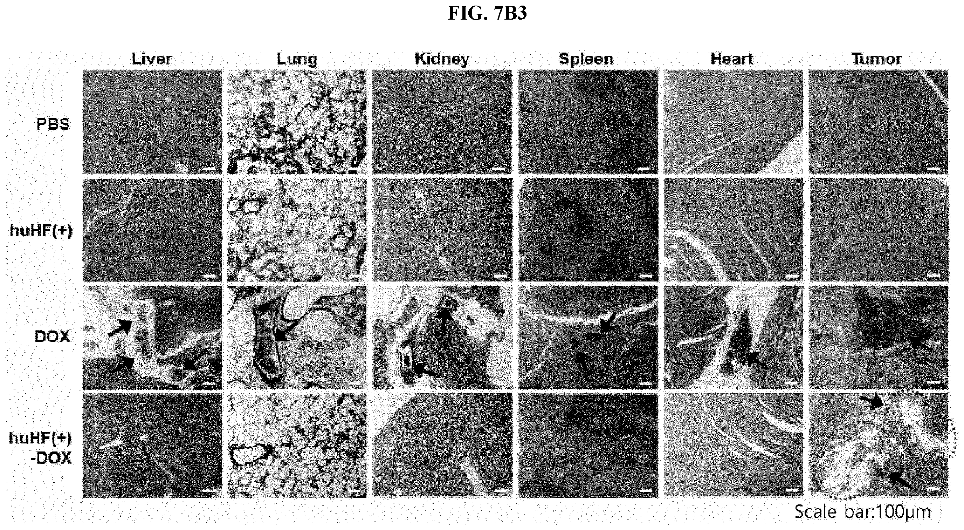

As shown in Examples 4 and 6, the protein nanoparticle huHF(+) exhibited a peptide on the surface, selectively inducing drug release by cathepsin E, so it was expected that it would not be toxic to normal cells, and as a result of testing toxicity to normal cells using cell lines of WI-38, IMR-90 and CCD-18co, it was verified that the toxicity to normal cells was significantly low. After 42 days of intravenous injection, liver, lung, kidney, spleen, heart and cancer of each experimental group were extracted and stained by H&E (hematoxylin & eosin), and each tissue was observed through an optical microscope (FIG. 7B). As a result, it was confirmed that when only anticancer drugs were administered, they were sporadically toxic to all organs and cancer. In contrast, when the protein nanoparticle-anticancer drug conjugates were administered, they were specifically toxic to cancer, and no toxicity-induced tissue damage was found in other organs.

## Claims

1. A ferritin fusion protein comprising:
a cancer-targeting peptide; and
a peptide to be cleaved in a cancer cell,
wherein the cancer-targeting peptide and the peptide to be cleaved in the cancer cell are linked to an outer surface of the ferritin.

2. The ferritin fusion protein of claim 1, wherein the peptide to be cleaved in the cancer cell is a peptide consisting of SEQ ID NO: 1.

3. The ferritin fusion protein of claim 1, further comprising a peptide configured for loading an anticancer drug linked to the peptide to be cleaved in the cancer cell.

4. The ferritin fusion protein of claim 3, wherein the peptide configured for loading the anticancer drug is a peptide comprising an amino acid sequence of DE.

5. The ferritin fusion protein of claim 4, wherein the peptide configured for loading the anticancer drug is an oligopeptide comprising the amino acid sequence of DE repeated 2 to 10 times.

6. The ferritin fusion protein of claim 1, wherein the cancer-targeting peptide has SEQ ID NO: 2.

7. The ferritin fusion protein of claim 1, wherein the ferritin consists of human ferritin heavy chains.

8. The ferritin fusion protein of claim 1, wherein the cancer-targeting peptide and the peptide to be cleaved in the cancer cell are independently linked to N-terminus or C-terminus of the ferritin monomer.

9. The ferritin fusion protein of claim 1, wherein the ferritin is a globular protein consisting of 24 ferritin monomers by self-assembly.

10. A pharmaceutical composition for preventing or treating a cancer, comprising:
the ferritin fusion protein of any one of claims 1 to 9; and
an anticancer drug linked to the peptide to be cleaved in the cancer cell of the ferritin fusion protein.

11. The pharmaceutical composition of claim 10, wherein the anticancer drug is linked to the peptide to be cleaved in the cancer cell via the peptide configured for loading the anticancer drug.

12. The pharmaceutical composition of claim 10, wherein the anticancer drug is a compound having anticancer activity.

13. The pharmaceutical composition of claim 10, wherein the anticancer drug is selected from the group consisting of gemcitabine, mitomycin C, doxorubicin, methotrexate, bleomycin, melphalan, chlorambucil, dacarbazine, cytarabine, fludarabine, pemetrexed, dactinomycin, daunorubicin, idarubicin, cladribine, hydroxycarbamide, thioguanine, bendamustine, temozolomide, azacitidine, clofarabine, decitabine, nelarabine, pralatrexate, epirubicin, eribulin, bexarotene, buserelin, crizotinib, dabrafenib, degarelix, goserelin, ibrutinib, lanreotide, lenvatinib, leuprorelin, mifamurtide, niraparib, pazopanib and raltitrexed.

14. The pharmaceutical composition of claim 10, wherein the cancer is selected from the group consisting of brain cancer, head and neck cancer, bladder cancer, breast cancer, cervical cancer, colon cancer, colorectal cancer, endometrial cancer, esophageal cancer, leukemia, lung cancer, liver cancer, ovarian cancer, pancreatic cancer, prostate cancer, rectal cancer, kidney cancer, gastric cancer, testicular cancer, uterine cancer, vascular tumor, squamous cell carcinoma, adenocarcinoma, small cell carcinoma, melanoma, glioma, neuroblastoma, sarcoma, laryngeal cancer, parotid cancer, biliary tract cancer, thyroid cancer, actinic keratosis, acute lymphocytic leukemia, acute myeloid leukemia, adenoid cystic carcinoma, adenoma, adenoid squamous cell carcinoma, anal canal cancer, anal cancer, anorectal cancer, astrocytoma, ganglion adenocarcinoma, basal cell carcinoma, bile cancer, bone cancer, bone marrow cancer, bronchial cancer, bronchial carcinoma, carcinoid, cholangiocarcinoma, chronic lymphocytic leukemia, chronic myelogenous leukemia, clear cell carcinoma, connective tissue cancer, cystic adenoma, digestive system cancer, duodenal cancer, endocrine system cancer, endoderm sinus tumor, endometrial hyperplasia, endometrial adenocarcinoma, endothelial cell carcinoma, ependymal cell, epithelial cell carcinoma, orbital cancer, focal nodular hyperplasia, gallbladder cancer, palpebral cancer, gastrobasal cancer, gastrinoma, glioblastoma, glucagonoma, heart cancer, hemangioblastoma, hemangioendothelioma, hemangioma, hepatodenoma, liver adenoma, hepatobiliary cancer, hepatocellular carcinoma, Hodgkin's disease, ileal cancer, insulinoma, intraepithelial neoplasia, intraepithelial squamous cell neoplasia, intrahepatic biliary tract cancer, invasive squamous cell carcinoma, jejunum cancer, joint cancer, pelvic cancer, giant cell carcinoma, colon cancer, lymphoma, malignant mesothelial cell tumor, medulloblastoma, medullary epithelioma, meningeal cancer, mesothelial cancer, metastatic carcinoma, oral cancer, mucoepithelial carcinoma, multiple myeloma, muscle cancer, nasal duct cancer, nervous system cancer, non-epithelial skin cancer, non-Hodgkin's lymphoma, soft cell carcinoma, oligodendroglioma, oral cancer, osteosarcoma, papillary serous adenocarcinoma, penile cancer, pharyngeal cancer, pituitary tumor, plasmacytoma, pseudosarcoma, pulmonary blastoma, rectal cancer, renal cell carcinoma, respiratory system cancer, retina blastoma, serous carcinoma, sinus cancer, skin cancer, small cell carcinoma, small intestine cancer, smooth muscle cancer, soft tissue cancer, somatostatin-secreting tumor, spine cancer, squamous cell carcinoma, striatal muscle cancer, submesothelial carcinoma, T cell leukemia, tongue cancer, ureter cancer, urethral cancer, cervical cancer, uterine trunk cancer, vaginal cancer, VIPoma, vulvar cancer, highly differentiated carcinoma, and Wilm's tumor.
